Europäisches Patentamt

**(19)** European Patent Office

Office européen des brevets

**(11)** **EP 0 807 685 A2**

**(12)** **EUROPEAN PATENT APPLICATION**

**(43)** Date of publication:
**19.11.1997 Bulletin 1997/47**

**(51)** Int. Cl.$^6$: **C12N 15/54**, C12N 9/10

**(21)** Application number: **97201062.3**

**(22)** Date of filing: **20.07.1990**

**(84)** Designated Contracting States:
**AT BE CH DE DK ES FR GB GR IT LI LU NL SE**

**(62)** Document number(s) of the earlier application(s) in accordance with Art. 76 EPC:
**90402084.9 / 0 466 995**

**(71)** Applicant: **ROUSSEL-UCLAF**
**93230 Romainville (FR)**

**(72)** Inventors:
• **van Assche, Charles**
  **FR/ 13190 Allauch (FR)**
• **Lando, Danielle**
  **75020 Paris (FR)**
• **Bruneau, Jean-Michel**
  **FR/ 75020 Paris (FR)**

• **Voelker, Toni Alois**
  **Davis, CA 95616 (US)**
• **Gervais, Monica**
  **95320 Saint Leu La Foret (FR)**

**(74)** Representative:
**Tonnellier, Marie-José et al**
**Département des Brevets,**
**102, Route de Noisy**
**93235 Romainville Cédex (FR)**

Remarks:
This application was filed on 10 - 04 - 1997 as a divisional application to the application mentioned under INID code 62.

**(54)** **Sucrose phosphate synthase (sps), its process for preparation, its cdna and utilization of cdna to modify the expression if sps in the plant cells**

**(57)** Sucrose phosphate synthase (SPS), its process for preparation, its cDNA, and utilization of cDNA to modify the expression of SPS in the plant cells.

EP 0 807 685 A2

## Description

The present invention relates to the sucrose phosphate synthase (SPS), its process for preparation, its cDNA, and utilization of cDNA to modify the expression of SPS in the plant cells.

Difficulties in the purification of sucrose phosphate synthase (SPS) from plants have interferred with efforts to characterize this enzyme. SPS catalyses the formation of sucrose phosphate, the sucrose precursor molecule, from fructose-6 phosphate and UDP-glucose in photosynthetically active plant cells. Sucrose phosphatase then acts on the sucrose phosphate moiety, in an irreversible reaction, to remove the phosphate and to release sucrose ready to translocate from the mature leaf (source) to any tissue requiring photoassimilate (sink), especially growing tissues including young leaves, seeds, and roots.

Because SPS is considered a rate limiting enzyme in the pathway providing sucrose to growing tissue, the study of SPS and its activity is of special interest. In a recent publication, Walker, J. L. & Huber, S.C., Plant Phys. (1989) 89 : 518-524, the purification and preliminary characterization of spinach (Spinachia oleracea) SPS was reported. However, monoclonal antibodies specific to the spinach SPS were found to be non-reactive with all other plants tested, "closely related" and "relatively unrelated species", including corn (Zea maize), soybean (Glycine max), barley (Hordeum vulgare), and sugar beet (Beta vulgaris). Thus, additional purified sources of SPS enzyme are needed for effective characterization of this factor. Especially of interest is the characterization of the corn SPS because of its very high export rates, as compared for example, to SPS levels of activity as found in the leaves of soybean.

With the advent of biotechnology, the ability to modify various properties of plants, especially agronomically important crops, is of interest. In this regard, it would be useful to determine the coding sequence for an SPS gene to probe other crop sources, to use such coding sequences to prepare DNA expression constructs capable of directing the expression of the SPS gene in a plant cell and to express a DNA sequence encoding an SPS enzyme in a plant to measure the effects on crop yield due to the increased rate of sucrose translocation to growing tissues.

In a first embodiment, proteins having the sucrose phosphate synthase (SPS) activity, namely, a protein capable of catalyzing the formation of sucrose phosphate from fructose-6-phosphate and UDP-glucose substrates, are provided. Among the preferred proteins of this invention are such proteins obtainable from corn which are substantially free of other proteins.

By "protein" is meant any amino acid sequence, including a protein, polypeptide, or peptide fragment, whether obtained from plant or synthetic sources, which demonstrates the ability to catalyze the formation of sucrose phosphate. An SPS of this invention will include sequences which are modified, such as sequence which have been mutated, trucated, increased, contain codon substitutions as a result of the degeneracy of the DNA code, and the like as well as sequences which are partially or wholly artificially synthesized, so long as the synthetic sequence retains the characteristic SPS activity.

By "substantially free from other proteins" is meant that the protein has been partially purified away from proteins found in the plant cell. Such a protein of this invention will demonstrate a specific enzymatic activity of at least greater than 0,05, more preferably at least greater than at least 0,30, wherein specific enzymatic activity (sA) is measured in units which correspond to 1 $\mu m$ (micromole) of sucrose formed per minute per mg of protein at 37°C. In a more preferred embodiment, the protein will demonstrate even more improved sA and increased purification factors (see, Table 1).

The invention relates to the enzyme comprising a corn SPS having a molecular weight from about 110 to 130 kilodalton (kd) and a specific activity of greater than 0,05 U.

The invention relates more particularly to the enzyme comprising a corn SPS having a specific activity of about 25 U.

In order to obtain the nucleic acid sequences encoding the SPS, especially corn SPS, substantially purified SPS was required. As demonstrated more fully in the examples, corn SPS purified 500-fold was obtained in small quantities which were then ultimately used to obtain peptide sequence which in turn led to the determination of the cDNA sequence.

Among the preferred proteins of the invention are the proteins having the above definition with a molecular weight from about 110 to about 130 kd, having the form of a monomer, a dimer or a tetramer and their derivatives, comprising at least one peptide having the following amino acid sequence :

Thr-Trp-Ile-Lys
Tyr-Val-Val-Glu-Leu-Ala-Arg
Ser-Met-Pro-Pro-Ile-Trp-Ala-Glu-Val-Met-Arg
Leu-Arg-Pro-Asp-Gln-Asp-Tyr-Leu-Met-His-Ile-Ser-His-Arg
Trp-Ser-His-Asp-Gly-Ala-Arg

The invention also relates to a process to prepare proteins as above defined, characterized in that :

**a)** one extracts from parts containing SPS, preserved at low temperature, by grinding, centrifugation and filtration,

**b)** one increases the SPS rate of the extract so obtained by precipitation in an appropriate solvent, centrifugation and solubilization of the precipitate in a buffer solution.

**c)** one purifies the protein so obtained by chromatography and if desired,

**d)** one prepares the hybridomas, and monoclonal antibodies from an antigenic solution prepared from one of the preparations a, b, c,

**e)** one screens the hybridomas and raises monoclonal antibodies specifically directed against SPS,

**f)** one purifies the SPS so obtained with the monoclonal antibodies so prepared.

The invention more precisely relates to a process of preparation of corn SPS characterized in that :

**a)** one extracts from part of corn plants by grinding centrifugation and filtration,

**b)** one increases the SPS rate of the extract so obtained by precipitation in polyethyleneglycol (PEG), centrifugation and solubilization of the precipitate obtained in a buffer solution,

**c)** one purifies the protein obtained in low pressure anion exchange chromatography and in chromatography on heparin sepharose, then in anion exchange high performance chromatography,

**d)** one purifies the active pools by passage on 2 high performance chromatography columns, and if desired,

**e)** one prepares the hybridomas and monoclonal antibodies from an antigenic solution prepared from one preparation a, b, c,

**f)** one screens the hybridomas and raises the monoclonal antibodies specifically directed against SPS,

**g)** one purifies the SPS preparation with the monoclonal antibodies so obtained.

Preferably :

- corn is a corn Pioneer corn hybrid strain 3184,
- part of plants are leaves kept at low temperature by example between -50°C and -90°C,
- purification in the polyethyleneglycol is realized

   . first by precipitating at a final concentration in PEG about 6 %,
   . then by precipitating at a final concentration about 12 %.

The different chromatographies are realized in the following way :

   1st chromatography DEAE sepharose
   2nd chromatography heparin sepharose : at this stage, the preparation obtained may be kept several days without loss of activity
   3rd chromatography Mono q chromatography
   4th chromatography HPLC hydroxyapatite
   5th chromatography HPLC hydroxyapatite.

- during the different steps of purification and thereafter, the SPS activity may be measured according two methods,

   **a)** a method based on a colorimetric test or resorcinol test,
   **b)** a method based on the dosage of one of the products formed during the transformation reactions where SPS is involved.

   Both methods are detailed in the experimental part detailed hereunder.
   Mice are immunized with several injections of enzymatic preparations.
   Different kinds of mice may be used, for example BALB/c.
   The antigen may be used in completed Freund adjuvant then in incompleted Freund adjuvant. Several injections in mice are realized : good results have been obtained with three injections of Mono q, pools, followed by three injections of final pools (days 0, 14, 27, 60, 90 and 105 for example).
   The first injections are administered sub-cutaneously, for example in the cushions, and the feet, the last injection is administered intravenously in the tail for example.
- the preparation of spleen cellular suspensions so immunized is made in a conventional way.

The steps of fusion with myelona cells, of conservation of the hybridoma, of cloning, of antibodies production are made by conventional ways.

To detect the hybridoma secreting the monoclonal antibodies raised against the antigen, two methods are used to select antibodies :

. a method of detection of antibodies as inhibitor of SPS activity,
. a method of detection of antibodies precipitating SPS activities.

In a preferred embodiment, these methods are the methods described in the experimental section detailed hereunder.

Among the objects of the invention, are also provided lines of hybridoma cells, and in particular hybridoma cells described as :

| SPA 2-2-3 : I-971 | SPB 3-2-19 : I-973 |
|---|---|
| SPA 2-2-22 : I-970 | SPB 5-2-10 : I-974 |
| SPA 2-2-25 : I-972 | SPB 5-4-2 : I-975 |
| | SPB 13-1-7 : I-976 |
| | SPB 13-2-2 : I-977 |

a deposit of which has been made at the C.N.C.M. ( INSTITUT PASTEUR PARIS ) on JUNE 11,1990.

The invention relates also to monoclonal antibodies specifically directed against SPS.

The invention relates also to a process of preparation of proteins as defined above characterized in that a preparation containing the socalled proteins is purified on a chromatography column having monoclonal antibodies as defined above specifically raised against the proteins.

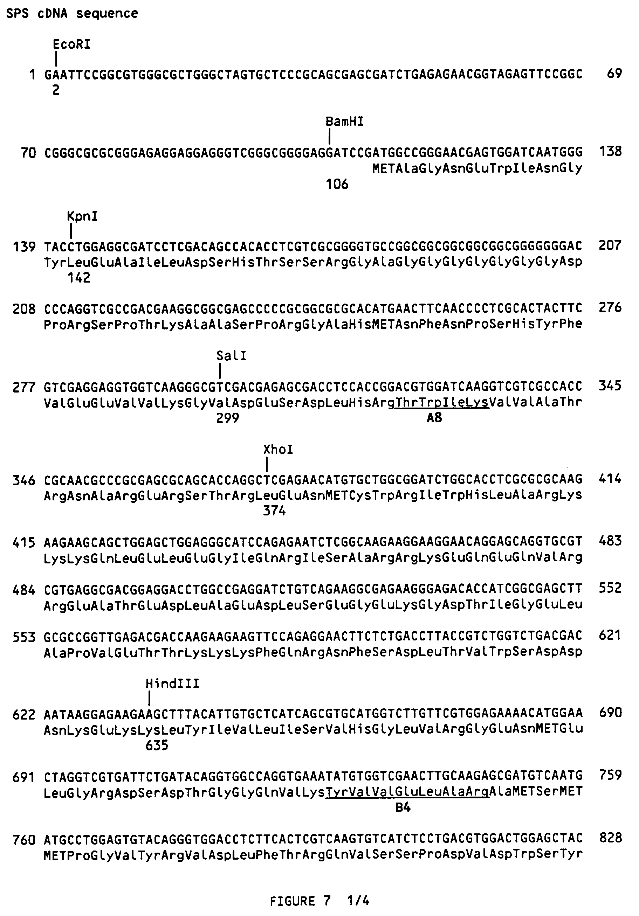

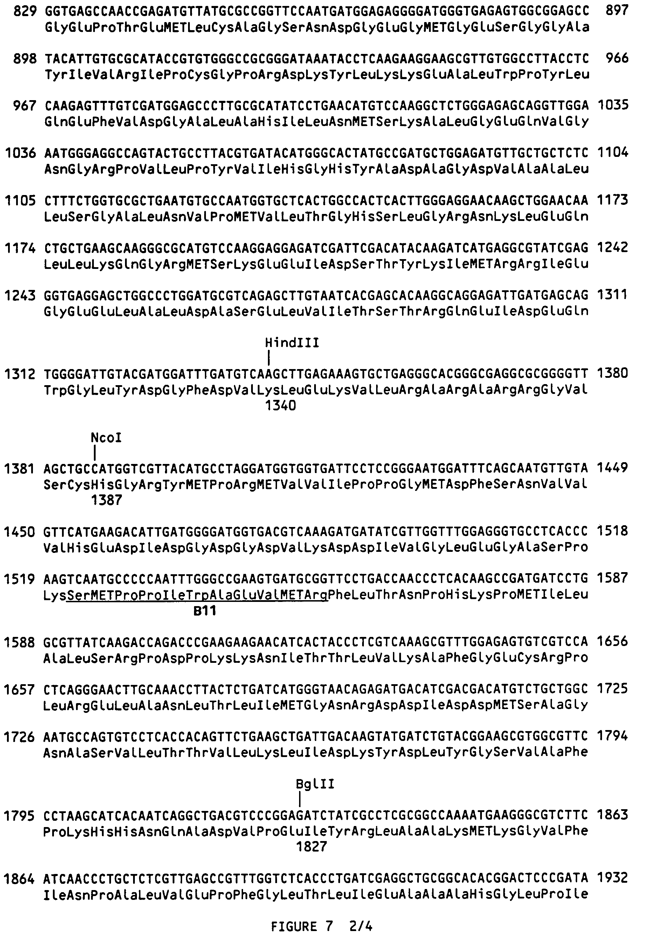

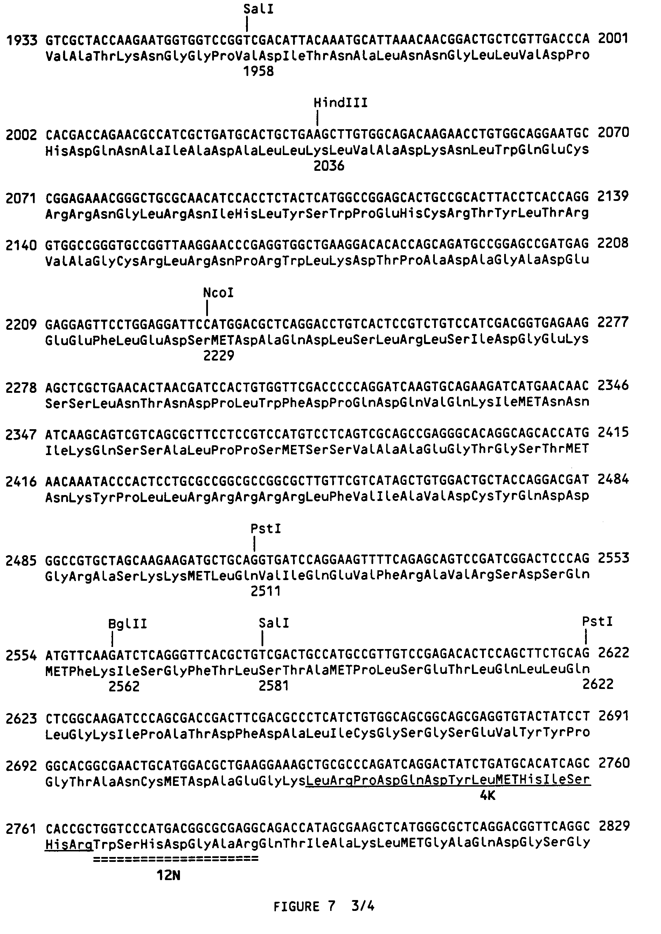

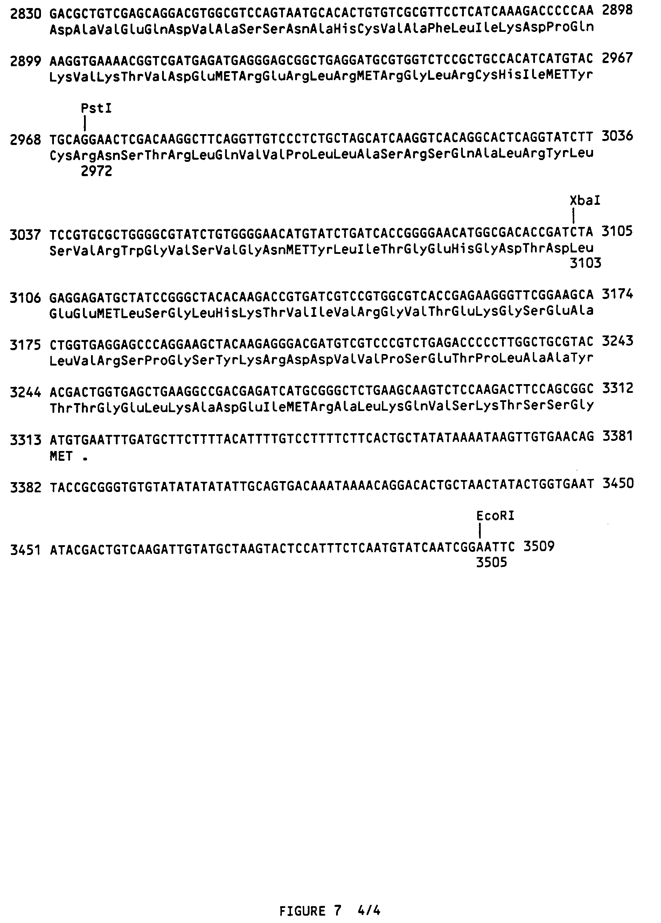

The invention relates also to cDNA coding for proteins as defined above, specially cDNA coding for corn SPS. Among the preferred cDNA preferred is the cDNA with the following nucleotide sequence represented in Figure 7.

Thus, this invention relates to an extrachromosomal DNA sequence encoding a SPS as defined above. Any DNA sequence which is not incorporated into the genome of a plant is considered extrachromosomal, i.e., outside of the chromosome, for purposes of this invention. This includes, but is not limited to cDNA, genomic DNA, truncated sequences, single stranded and double stranded DNA. In a preferred embodiment, the DNA sequence is cDNA. In a different preferred embodiment, the DNA sequence is obtainable from corn.

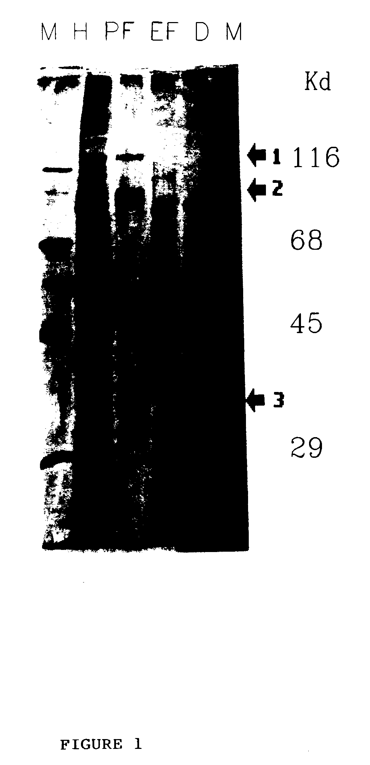

Among the preferred proteins and nucleic acid sequences of the invention is corn SPS. The corn SPS is represented in Figure 1, which shows the presents of proteins at about 120, 95 and 30 kd. The Proteins shown at 95 and 30 kd are considered to be breakdown products of the protein shown at 120 kd. The complete protein is believed to a a di- or tetrameric protein having as the basic sub-unit from about a 110 to about 130 kd protein. The complete cDNA sequence of the corn SPS is shown in Figure 7.

cDNA coding for sucrose phosphate synthase has been prepared in the following way.

1) Sequencing of peptide fragments from purified SPS. With the purified preparations of SPS previously obtained, by separating on acrylamide gel, a 120 kd minor band (corresponding to the total protein sequence) and two 90 kd and 30 kd major bands are obtained. Both major polypeptides are separated on electrophoresis and electroeluted. By a trypsin digestion and the sequencing of fragments so obtained, the sequence of 5 peptides has been determined.

This aminoacid sequence allows to determine the corresponding degenerate nucleotide sequence.

2) Corn leaf isolation.

Total RNA is isolated according to TURPEN and GRIFFITH (1986, Biotechniques vol. 4 pages 11-15) for poly(A) RNA preparation, the standard oligo dT cellulose column was used.

3) cDNA library construction.

cDNA synthesis is realized by following the protocol of a kit supplied by PROMEGA except that M-MLV reverse transcriptase is used instead of AMV reverse transcriptase. The length of cDNA obtained is from 500 to several thousand base pairs. One adds ECORI linkers to the blunt ended cDNA and clones this material into a second generation lambda GT11 expression vector. Total library size is about $1,5.10^6$ plaques.

4) Utilization of PCR in order to synthesize a nucleotide sequence specific for SPS.

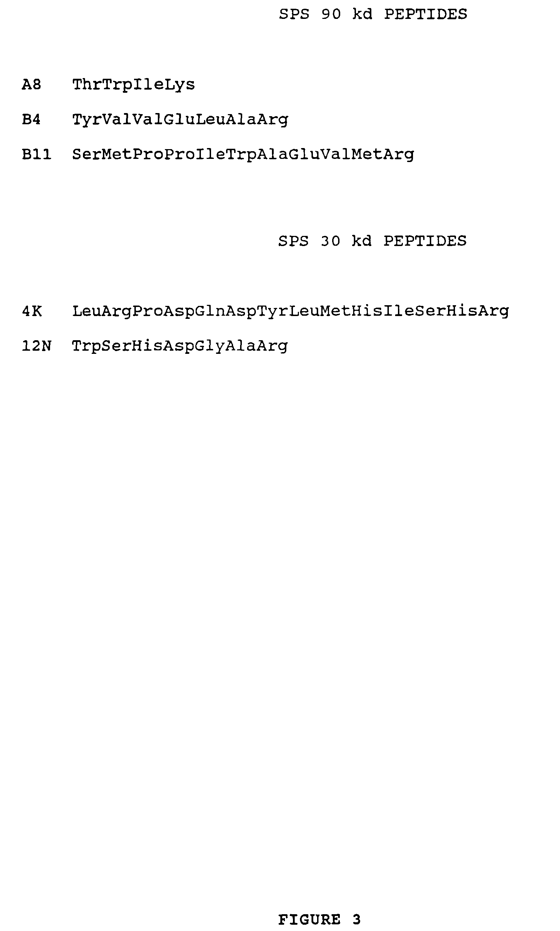

The oligonucleotides derived from peptides B11 (SPS 30 kd) and 4K (90 kd) described in figure 3 are used as primers in a PCR reaction. It has been assumed that peptides derived from SPS 30 and SPS 90 are degradation products of protein SPS 120 kd, and that, peptides derived from SPS and SPS 90 are encoded by the same RNA.

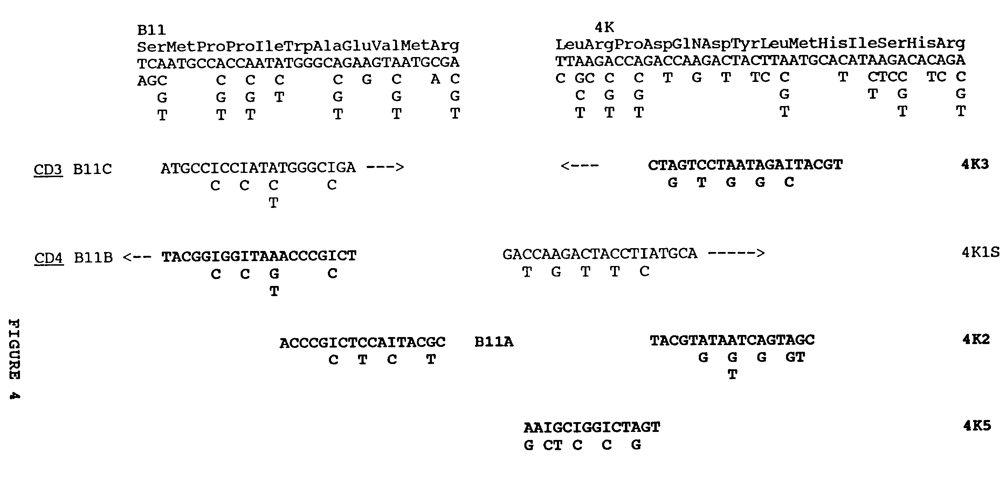

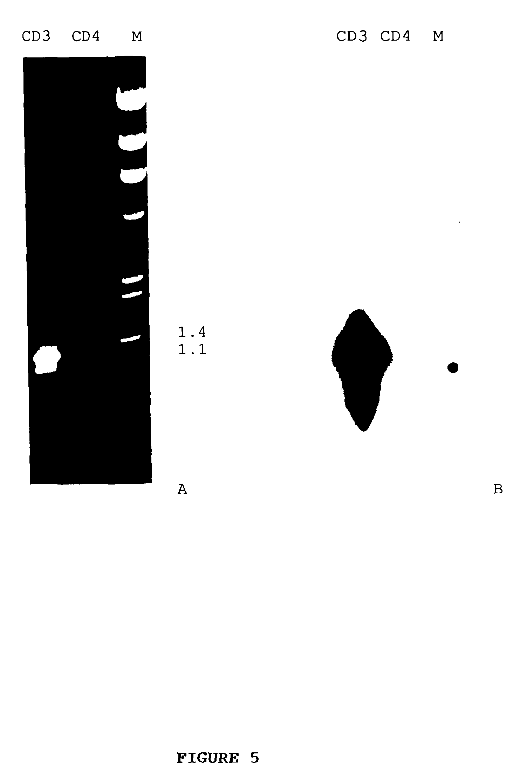

With this hypothesis, by using in proper polarity pairs of oligonucleotides corresponding to the peptidic sequences in a PCR reaction, one may obtain the synthesis of the DNA, connecting the two location. Since it is a priori not know in which order the peptides are located relative to each other, one has to do the two different possibilites Fig. 4. Only the oligonucleotide couple CD3 synthesizes a cDNA of defined length (1200 bp) (Fig. 5).

5) cDNA libray screening.

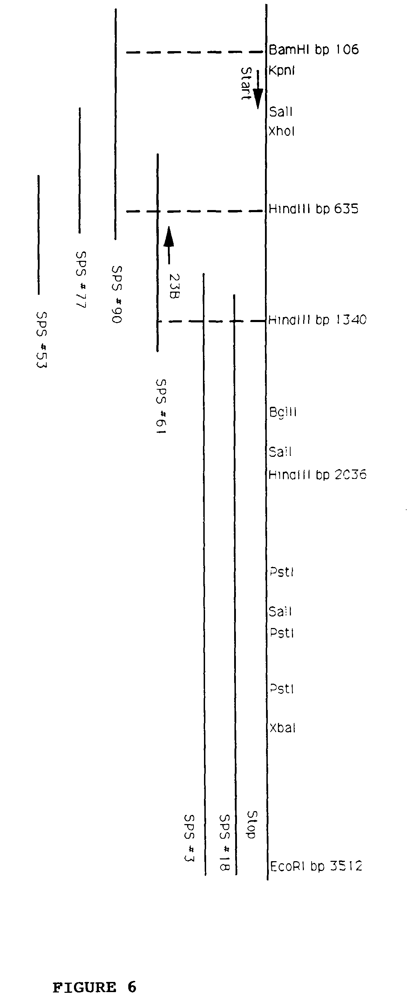

When 250000 lambda clones GT11 are screened using the 1200 bp long PCR cDNA, 16 positives are obtained. Sizes of the inserts ranged from 0,3 kb to 2,8 kb (see Fig. 6 for the two longest clones). The sequence is not complete in 5'. In a second round of library screening with a 400 bp DNA fragment corresponding to the most 5' fragment of the clone SPS 3, we obtain a SPS 61 clone extending further 5' without having the 5' end of the reading frame (Fig. 6).

6) Creation and screening of a second cDNA libray in order to clone the 5' sequence of cDNA coding for SPS.

A oligonucleotide complementary to the 5' sequence of clone SPS 61 is used as a primer for cDNA synthesis. After second strand reaction is completed, the cDNA is cloned into bacteriophage lambda GT11. The library includes about one million clones. The SPS 90 and SP 77 have been obtained, by screening this library with SPS 61 (Fig. 6).

7) The assembled SPS reading frame.

DNA sequences which encode the SPS may be employed as a gene of interest in a DNA construct or as probes in accordance with this invention. When found in a hoste cell, the sequence may be expressed as a source of SPS. More preferred is the SPS sequence in a vegetal cell under the regulating control of transcriptional and translational initiation region functional in plants.

Vegetal cell means any plant cell being able to form undifferentiated tissues as callus or differentiated tissues as embryos, parts of plants, whole plants or seeds.

Plants means for example plant producing grain seeds for example such as cereals, such as wheat, barley, corn, or oat, leguminous such as soybean, oleaginous as turnesol, tuber as potato, plan with roots as beet or fruit as tomato. The sucrose phosphate synthase is a key enzyme, in sucrose regulation mechanisms, but also in carbon partitioning regulation between starch and sucrose during photosynthesis (see Jack PREISS, TIBS January 1984, page 24, or Mark STITT and Coll, BIOCHEMISTRY of PLANTS, vol. 10, 1987, pages 3-27).

When found in a DNA construct for integration into a plant genome, the sequence may be found is a sense orientation or anti-sense orientation. By increasing the amount of SPS available to the photosynthetically active plant cell by the expression of additional SPS, an increased flow of sucrose may be provided to growing tissues, for example, resulting in increased plant yields ; by decreasing the amount of SPS available to the photosynthetically active plant cell, the rate of sucrose release from the plant cell may be hindered, resulting in less new plant growth.

By "obtainable from corn" is meant that the sequence, whether an amino acid sequence or nucleic acide sequence, is related to a corn SPS, including a SPS recovered through use of nucleic acid probes, antibody preparations, sequence comparisons or derivatives obtained through protein modeling or mutagenesis for example. Thus, one skilled in the art will readily recognize that antibody preparation, nucleic acid probes (DNA and RNA) and the like may be prepared and used to screen and recover other plant sources for SPS. Typically, a homologously related nucleic acid sequence will show at least about 60 % homology, and more preferably at least about 70 % homology between the corn SPS and the given plant SPS of interest, excluding any deletions which may be present. Homology is found when there is an identity of base pairs an may be determined upon comparison of sequence information, nucleic acid or amino acide, or through hybridization reactions conducted under relatively stringent conditions, e.g., having a fairly low percentage of non-specific binding with corn SPS probes.

Probes can be considerably shorter than the entire sequence, but should be at least about 10, preferably at least about 15, more preferably at least 20 nucleotides in length. Longer oligonucleotides are also useful, up to full length of the gene encoding the polypeptide of interest. Both DNA and RNA probes can be used.

A genomic library prepared from the plant source of interest may be probed with conserved sequences from corn SPS to identify homologously related sequences. Use of the entire corn SPS cDNA may be employed if shorter probe sequences are not identified. Positive clones are then analyzed by restriction enzyme digestion and/or sequencing. In this general manner, one or more sequences may be identified providing both the coding region, as well as the transcriptional regulatory elements of the SPS gene from such plant source.

In use, probes are typically labeled in a detectable manner (for example with $^{32}$P-labelled or biotinylated nucleotides) and are incubated with single-stranded DNA or RNA from the plant source in which the gene is sought, although unlabeled oligonucleotides are also useful. Hybridization is detected by means of the label after single-stranded and double-stranded (hybridized) DNA or DNA/RNA have been separated, typically using nitrocellulose paper or nylon membranes. Hybridization techniques suitable for use with oligonucleotides are well known to those skilled in the art.

From cDNA sequences, one skilled in the art will be readily able to obtain the corresponding genomic DNA sequences related thereto to obtain the coding region of the SPS including intron sequences, transcription, translation initiation regions and/or transcript termination regions of the respective SPS gene. The regulatory regions may be used with or without the SPS gene in various probes and/or constructs.

The complete SPS reading frame can be assembled using restriction enzyme fragments of SPS 90, SPS 61 and SPS 3, see Fig. 6.

When expressed in E. Coli, the SPS cDNA produces a protein which is recognized by anti SPS antisera and has the same electrophoretic mobility as SPS extracted from corn leaves. We show that this E. Coli SPS is as active as plant SPS, i.e. for complete enzymatic activity in E. Coli no other plant factor is needed but the SPS cDNA.

Plants obtained by the method of transformation and contain fusions of SPS cDNA to tissue specific promoters in order to modify or alter the composition of certain plant organs is also included.

A DNA construct of this invention may include transcriptional and translational initiation regulatory regions homologous or heterologous to the plant host. Of particular interest are transcriptional initiation regions from genes which are present in the plant host species, for example, the tobacco ribulose biphosphate carboxylase small subunit (ssu) transcriptional initiation region ; the cauliflower mosaic virus (CaMV) 35S transcriptional initiation region, including a "double" 35S CaMV promoter ; and those associated with T-DNA, such as the opine synthase transcriptional initiation region, e.g., octopine, mannopine, agropine, and the like.

Any one of number of regulatory sequences may be preferred in a particular situation, depending upon whether constitutive or tissue and or timing induced transcription is desired, the particular efficiency of the promoter in conjunction with the heterologous SPS, the ability to join a strong promoter with a control region from a different promoter which allows of inducible transcription, ease of construction and the like. For example, tissue specific promoters may be employed to selectively modify or alter the composition of certain plant organs. These regulatory regions find ample precedence in the literature.

The termination region may be derived from the 3'-region of the gene from which the initiation region was obtained, from the SPS gene, or from a different gene. Preferably the termination region will be derived from a plant gene, particularly, the tobacco ribulose biphosphate carboxylase small subunit termination region ; a gene associated with the Ti-plasmid such as the octopine synthase termination region or the tml termination region.

In developing the expression cassette, the various fragments comprising the regulatory regions and open reading frame may be subjected to different processing conditions, such a ligation, restriction, resection, in vitro mutagenesis, primer repair, use of linkers and adapters, and the like. Thus, nucleotide transitions, transversions, insertions, deletions, or the like, may be performed on the DNA which is employed in the regulatory regions and/or open reading frame.

During the construction of the expression cassette, the various fragments of the DNA will usually be cloned in an appropriate cloning vector, which allows for amplification of the DNA, modification of the DNA or manipulation by joining or removing of the sequences, linkers, or the like. Normally, the vectors will be capable of replication in at least a relatively high copy number in E. Coli. A number of vectors are readily available for cloning, including such vectors as pBR322, pUC series, M13 series, etc. The cloning vector will have one or more markers which provide for selection or transformants. The markers will normally provide for resistance to cytotoxic agents such as antibiotics, heavy metals, toxins, or the like. By appropriate restriction of the vector and cassette, and as appropriate, modification of the ends, by chewing back or filling in overhangs, to provide for blunt ends, by addition of linkers, by tailing, complementary ends can be provided for ligation and joining of the vector to the expression cassette or component thereof.

After each manipulation of the DNA in the development of the cassette, the plasmid will be cloned and isolated and, as required, the particular cassette component analyzed as to its sequence to ensure that the proper sequence has been obtained. Depending upon the nature of the manipulation, the desired sequence may be excised from the plasmid and introduced into a different vector or the plasmid may be restricted and the expression cassette component manipulated, as appropriate.

The manner of transformation of E. Coli with the various DNA constructs (plasmids and viruses) for cloning is not critical to this invention. Conjugation, transduction, transfection or transformation, for example, calcium phosphate mediated transformation, may be employed.

In addition to the expression cassette, depending upon the manner of introduction of the expression cassette into the plant cell, other DNA sequences may be required. For example when using the Ti- or Ri-plasmid for transformation of plant cells, as described below, at least the right border and frequently both the right and left borders of the T-DNA of the Ti- or Ri-plasmids will be joined as flanking regions to the expression cassette. The use of T-DNA for transformation of plant cells has received extensive study and is amply described in Genetic Engineering, Principles and Methods (1984) Vol 6 (Eds. Setlow and Hollaender) pp. 253-278 (Plenum, NY) ; A. Hoekema, in : The Binary Plant Vector System (1985) Offsetdrukkerij Kanters, B.V. Alblasserdam.

Alternatively, to enhance integration into the plant genome, terminal repeats of transposons may be used as borders in conjunction with a transposase. In this situation, expression of the transposase should be inducible, so that once the expression cassette is integrated into the genome, it should be relatively stably integrated and avoid hopping.

The expression cassette will normally be joined to a marker for selection in plant cells. Conveniently, the marker may be resistance to a biocide, particularly an antibiotic, such as Kanamycin, G418, Bleomycin, Hygromycin, Chloramphenicol, or the like. The particular marker employed will be one which will allow for selection of transformed plant cells as compared to plant cells lacking the DNA which has been introduced.

A variety of techniques are available for the introduction of DNA into a plant cell host. These techniques include transformation with Ti-DNA employing A. tumefaciens or A. rhizogenes as the transforming agent, protoplast fusion, injection, electroporation, DNA particle bombardment, and the like. For transformation with agrobacterium, plasmids can be prepared in E. coli which plasmids contain DNA homologous with the Ti-plasmid, particularly T-DNA. The plasmid may be capable of replication in Agrobacterium, by inclusion of a broad spectrum prokaryotic replication system, for example RK290, if it is desired to retain the expression cassette on a independent plasmid rather than having it integrated into the Ti-plasmid. By means of a helper plasmid, the expression cassette may be transferred to the A. tumefaciens and the resulting transformed organism used for transforming plant cells.

Conveniently, explants may be cultivated with the A. tumefaciens or A. rhizogenes to allow for transfer of the expression cassette to the plant cells, the plant cells dispersed in an appropriate selection medium. The Agrobacterium host will contain a plasmid having the vir genes necessary for transfer.

After transformation, the cell tissue (for example protoplasts, explants or cotyledons) is transferred to a regeneration medium, such as Murashige-Skoog (MS) medium for plant tissue and cell culture, for formation of a callus. Cells which have been transformed may be grown into plants in accordance with conventional ways. See, for example, McCormick et al., Plant Cell Reports (1986) 5 : 81-84. The transformed plants may then be analyzed to determine whether the desired gene product is still being produced in all or a portion of the plant cells. After expression of the desired product has been demonstrated in the plant, the plant can be grown, and either pollinated with the same transformed strain or different strains and the resulting hybrid having the desired phenotypic characteristic identified. Two or more generations may be grown to ensure that the subject phenotypic characteristic is stably maintained and inherited.

## 1 - PURIFICATION OF SUCROSE PHOSPHATE SYNTHASE OF CORN

### 1.1 - Method of determination of enzymatic activity (SPS)

During purification SPS activity is followed in 2 ways :

**a)** either by means of a colorimetric test (P.S. Kerr et al., Planta., 1987, 170 : 515-519) called resorcinol test described below.
Sucrose Phosphate Synthase or UDP glucose - D Fructose - Phosphate Glucosyltransferase (EC 2.4.1.14) catalyzes the reaction :

$$\text{UDPG} + \text{Fructose 6-P} \Leftrightarrow \text{Sucrose 6-P} + \text{UDP}$$

| | |
|---|---|
| UDPG | : Uridine Di-Phospho Glucose |
| Fructose 6-P or F6P | : Fructose 6-Phosphate |
| Sucrose 6-P | : Sucrose 6-Phosphate |

The sucrose 6-P formed reacts with the Resorcinol to give a red-colored compound quantifiable by spectrophotometry at 520 nm (nanometer) (Optical Density (O.D.) = 520 nm). In practice, to 45 $\mu$l (microliter) of enzymatic preparation 25 $\mu$l of a buffered solution containing the two substrates is added (UDPG 70 mM, F6P 28 mM, MgCl$_2$ 15 mM, HEPES 25 mM pH 7,5). After incubation at 37°C, reaction is stopped by adding 70 $\mu$l of NaOH in solution and heating at 95°C during 10 mn. After cooling, 0,25 ml of a solution 0,1 % resorcinol in ethanol 95 % is added, then 0,75 ml of HCl 30 % is added. The OD 520 mM is read after incubation 8 mn at 80 mn, and cooling.
**b)** or by means of a coupled enzymatic system (S. Harbron et al., Anal. Biochem. 1980, 107 : 56-59) being composed in the following way :

$$\text{UDPG} + \text{F6P} \Leftrightarrow \text{Sucrose 6 P} + \text{UDP}$$

SPS

$$\text{UDP} + \text{ATP} \Leftrightarrow \text{ADP} + \text{UTP}$$

Nucleoside Diphosphokinase NP$_2$K

$$\text{ADP} + \text{PEP} \Leftrightarrow \text{Pyruvate} + \text{ATP}$$

Pyruvate kinase PK

$$\text{Pyruvate} + \text{NADH} \Leftrightarrow \text{NAD} + \text{lactate}$$

## Lactate dehydrogenase LDH

The disappearance of the NADH absorption at 340 nm is monitored 1 mole of NAD formed or 1 mole of NADH consumed corresponds to 1 mole of sucrose 6 P formed.

In pratice, in a quartz spectrophotometric tun thermostated at 37°C, the following solution are added.

- 540 $\mu$l of HEPES buffered 50 mM, $MgCl_2$ 10 mM KCl 20 mM pH=7.5,
- 250 $\mu$l of a mixture of substrates PEP (1,6 mM NaDH 0,6 mM, ATP 4 mM UDPG 112 mM),
- 60 $\mu$l of an enzyme mixture (LDH 166,7 U/ml PK 333,3 U/ml, NPzK 66,7 U/ml),
- 100 $\mu$l of F6P 112 mM.

After homogenization, 50 $\mu$l of the preparation containing SPS is added, the diminution of optical density at 340 nm is added with a spectrophotometer (UVIKON 860, KONTRON instruments). The measure is done with the kinetic of the machine.

### 1.2 - Purification of the SPS (preparation of the immunogen)

#### 1.2.1 - Extraction

The starting material for the purification are mature leaves of young corn plants (Zea mays L. cv Pioneer 3184), which have been harvested in late morning, cut up, deveined, frozen in liquid nitrogen and stored at -70°C.

250 g of leaves are suspended in 1 liter of 50 mM HEPES 10 mM $MgCl_2$ 1 mM EDTA 5 mM DTT, pH=7.5 buffer (extraction buffer) which has observed to it 11 g of Polyvinyl-pyrrolidone, nitrogen is bubbled through and the suspension is cooled to 0°C.

The leaves are ground, until a homogeneous liquid is obtained. This ground product is filtered, and then centrifuged at 14,000 g for 20 minutes at 4°C.

While the bubbling through of nitrogen is maintained, a solution of 50 % Poly Ethylene Glycol (PEG 8000 "Breox" at 50 % w/v of extraction buffer) is added to the supernatant until a final concentration of PEG of 6 % is reached. Then the suspension is cooled at 0°C. After centrifuging at 14,000 g for 20 minutes the supernatant has added to it 50 % PEG until a final concentration of PEG of 12 % is reached. After a repeated centrifugation, the supernatant is discarded and the residue is solubilized with 60 ml of 50 mM HEPES, 10 mM $MgCl_2$, 1 mM EDTA, 5 mM DTT, 10 % Ethylene Glycol (EG), 0.08 M KCl, pH 7.5 buffer (recovery buffer). This solution is clarified by centrifuging at 40,000 g for 10 minutes. The supernatant constitutes the final extract.

#### 1.2.2 - Low pressure anion-exchange chromatography : fast-flow DEAE Sepharose exchanger

The final extract is chromatographed on a column 25 mm x 162 mm of 80 ml of Fast-Flow DEAE Sepharose PHARMACIA equilibrated with recovery buffer. After washing the column with the same buffer, the proteins adsorbed on the support are eluted by means of a linear gradient with increasing ionic strength between 0.08 M KCl and 0.35 M KCl in the 50 mM HEPES, 10 mM $MgCl_2$, 1 mM EDTA, 5 mM DTT, 10 % EG, pH 7.5 buffer (buffer A). The flow rate applied during this experiment is 180 ml/h and chromatography is executed at 4°C.

The SPS activity is eluted at about 0.17 M KCl.

#### 1.2.3 - Chromatography on heparin Sepharose

The fractions containing the SPS activity are collected and diluted to one fifth in buffer A, then added to 12 ml of heparin Sepharose previously equilibrated with buffer A. After one hour of incubation with gentle agitation at 4°C, the gel is washed with about 10 volumes of buffer A + 0.05 M KCl, then repacked in a chromatography column.

The proteins adsorbed are eluted in an isocratic way by means of a 10 mM CAPS, 10 mM $MgCl_2$, 1 mM EDTA, 5 mM DTT, 10 % EG, 0.01 % Tween 80, 1 mg/ml heparin, 1 % Fructose, 0.25 M KCl, pH 10 buffer, delivered at 60 ml/h.

Chromatography is executed at 4°C.

The fractions containing the SPS activity are collected (heparin fraction) and preserved on ice until the following purification stage. The enzyme at this stage is stable for a least one week.

The following purification steps are carried out using a system of High Performance Liquid Chromatography (HPLC) ; the purification is followed by means of a detector fitted with a filter enabling absorbency in the ultra-violet at 280 nm (A280) to be measured. The buffers and the fractions recovered are kept at low temperature.

### 1.2.4 - **High performance anion-exchange chromatography : Mono Q**

The heparin fraction is diluted by adding one third volume of 20 mM Triethanolamine, 10 mM $MgCl_2$, 1 mM EDTA, 10 mM DTT, 3 % EG, 0.3 % Tween 80, pH 7.5 buffer (buffer A) and loaded on an FPLC Mono Q HR10/10 column, (10 x 100 mm PHARMACIA) previously equilibrated with the same buffer which has added to it NaCl (final concentration 0.18 M). After the A280 has returned to 0, the proteins adsorbed on the chromatography support are eluted by means of a salt-complex gradient comprised as follows :

buffer A :       cf above
buffer B :       buffer A + NaCl 1 M

| time (minutes) | % B |
| --- | --- |
| 0 | 18 |
| 0.1 | 24 |
| 15 | 24 |
| 19 | 26 |
| 23 | 26 |
| 33 | 31 |
| 38 | 31 |
| 41 | 100 |
| 43 | 18 |

The flow rate applied to the column is 180 ml/h.
The SPS activity is eluted between 0.26 and 0.31 M NaCl.
The active fractions are collected together ("Mono Q fraction").

### 1.2.5 - **HPLC on Hydroxyapatite**

The Mono Q fraction is loaded on an HPLC column of hydroxyapatite 4 mm x 75 mm neutralized with 20 mM $KH_2PO_4/K_2HPO_4$, 3 % EG, 0.3 % Tween 80, 5 mM DTT, pH 7.5 buffer. After the A280 has returned to 0, the proteins adsorbed are eluted by means of the following phosphate gradient :

buffer A :       cf above
buffer B :       idem buffer A but 500 mM Phosphate of K

| time (minutes) | % B |
|---|---|
| 0 | 2 |
| 5 | 11 |
| 9 | 13 |
| 14 | 13 |
| 29 | 40 |
| 31 | 100 |
| 32 | 100 |
| 35 | 2 |

The flow rate applied is 60 ml/h. At this stage, the phosphate will partially inhibit SPS activity and therefore it is difficult to calculate a specific activity and also a purification factor (cf table 1) at this stage.

The SPS activity is eluted under these conditions with about 60 mM phosphate.

The active fractions are collected together and constitute the HAC fraction.

### 1.2.6 - **HPLC on DEAE 5PW**

The HAC fraction is loaded on an anion-exchange HPLC column of Di Ethyl Amino Ethyl type (DEAE-5PW) previously neutralized with a buffer of 20 mM Triethanolamine, 10 mM $MgCl_2$, 1 mM EDTA, 3 % EG, 2.5 mM DTT, 2 % betaine, pH 7.5 buffer (buffer A) + 0.15 M NaCl.

After the A280 has returned to 0, the proteins adsorbed are eluted by means of the following NaCl gradient :

buffer A :    cf above
buffer B :    idem buffer A with 1 M NaCl

| time (minutes) | % B |
|---|---|
| 0 | 15 |
| 0.1 | 20 |
| 5 | 20 |
| 22 | 35 |
| 27 | 35 |
| 30 | 100 |
| 31 | 15 |

The flow rate used is 60 ml/h.

The SPS activity is eluted with about 0.3M NaCl.

### 1.2.7 - **Preparation of the final preparation : concentration**

The final preparation is concentrated by HPLC chromatography on Mono Q HR5/5 exchanger (5 X 50 mm, Pharmacia) and rapid elution.

The DEAE 5PW fraction (or the G200 fraction) is diluted to two thirds with buffer A (idem 6) and loaded on the column previously neutralized with buffer A + 0.18 M NaCl. The following gradient is then applied on the column :

buffers A and B :     idem 6

| time (minutes) | % B |
|---|---|
| 0 | 18 |
| 10 | 40 |
| 12 | 100 |
| 13 | 18 |

The flow rate used is 60 ml/h.

The SPS activity is eluted with about 0.3 M NaCl.

The final preparation is stored at -20°C until used. Table 1 summarizes the results obtained at the various purification stages in terms of quantities of proteins recovered and of SPS activity.

TABLE 1

| | Concentration of proteins (mg/ml) | Volume (ml) | sA** (U) | pF** | Y* (%) |
|---|---|---|---|---|---|
| Ground product | 1 | 1000 | 0.05 | 0 | 100 |
| Final extract | 4< <8 | 60 | 0.30 | 6 | 144 |
| DEAE FF fraction | 0.4< <0.8 | 70 | 3 | 60 | 168 |
| Heparin fraction | 0.2< <0.4 | 25 | 9 | 180 | 90 |
| Mono Q fraction | (0.02) | 30 | - | - | - |
| HAC fraction | (0.03) | 8 | - | - | - |
| Final preparation | 0.05 | 2 | 25 | 500 | 5 |

Key
sA = Specific enzymatic activity : 1 U corresponds to 1 μm of sucrose formed per minute per mg of protein at 37°C
pF = Purification factor
Y = Yield
( ) = approximate value
- = not determined

Observations :
** the measurement of the quantity of proteins is carried out using the Bradford method. As Tween interferes enormously with this method, it is not possible to determine the proteins and then to calculate an sA at the level of the stages containing one. Furthermore, as phosphate is an inhibitor of SPS activity, the determination during the HAC stage gives an underestimated result.
* the increasing yield during the initial stages can be explained by the elimination, during purification, of certain inhibitors of SPS activity.

An SPS-PAGE profile at various stages of the purification process and the quality of the final preparation is given in Figure 1. The 120, 95 and 35 kd proteins are correlated to the SPS activity.

The 35 and 95 kd proteins are very likely breakdown products of the 120 kd protein as it can be shown by the nucleotidic sequence coding for the SPS protein.

Furthermore, the antibodies directed against the 35 and 95 kd proteins also recognize the protein 120 kd in immunodetection after membrane transfer, which demonstrates an antigenic identity between these three proteins (see below). It must be pointed out, however, that the addition of protease inhibitors in the buffers during purification has not enabled us to obtain a single 120 kd protein.

Gel permeation chromatographies were carried out in order to find the apparent molecular weight of the native SPS protein. Briefly, the HAC fraction is concentrated by HPLC chromatography on Mono Q HR 5/5 inchanger (see 1-2-7). The active fractions are collected together (about 2 ml) and loaded on an G 200 column previously washed with a buffer containing 20 mM triethanolamin, 10 mM $MgCl_2$, 1 mM EDTA, 3 % E.G., 2,5 mM DTT, 2 % betaïn, 0,3 M NaCl pH 7,5. The SPS activity is eluted a major protein peak corresponding to an apparent mass of 270-280 kda which is in agreement with the results obtained by S. HARBRON an al. (Arch. Biochem. Biophys., 1981, 212 : 237-246) with the spinach SPS. It can be noted that the chromatography on a TS lambda 60000 permeation column lead to the elution of the SPS activity at a retention time corresponding to an apparent mass of 440 kda which is near from the value obtained by DC. DOEHLERT and S.C. HUBER (Plant Physiol., 1983, 73 : 989-994) with the spinach SPS, using a AcA34 permeation column.

The SPS protein seems therefore to be a di or tetrameric protein having as the basic sub-unit a 120 kda protein (homo-dimeric or homo-tetrameric).

<u>Key for Figure 1</u>

SDS-PAGE profile of sucrose phosphate synthase of corn : 8.5 % acrylamide gel, reducing conditions and staining with silver nitrate
M : Standard of molecular weight B-Galactosidase (116 kd), bovine Albumin (68 kd), Egg Albumin (45 kd), carbonic Anhydrase (29 kd).
H : Heparin fraction, 30 micrograms of proteins per well.
FP : Final Preparation, 7.5 micrograms of proteins per well.
FE : Final Extract, 7.5 micrograms of proteins per well.
D : Fast-Flow DEAE fraction, 7.5 micrograms of proteins per well.

The bands of proteins visible at about 120 kd (1), 95 kd (2) and 35 kd (3) are correlated, during the chromatography stages, with the appearance of SPS activity in the respective fractions.

## 2 - **PROCESS FOR THE PREPARATION OF MONOCLONAL ANTIBODIES DIRECTED AGAINST SUCROSE PHOSPHATE SYNTHASE**

### 2.1 - <u>Immunisations</u>

BALB/C mice are immunized by subcutaneous injection (pads and paws) according to the following methodology :

Day 0 injection of about 5 micrograms of proteins (or about 0.3 U SPS per mouse) : Mono Q pool emulsified volume for volume with Freund's Complete Adjuvant (FCA).

Day 14 injection of about 5 micrograms of proteins (or about 0.3 U SPS per mouse) : Mono Q pool emulsified volume for volume with Freund's Incomplete Adjuvant (FIA).

Day 27 Idem D14

Day 0 + 60 injection of about 20 micrograms of proteins : final pool in FIA

Pay 0 + 90 injection of about 12 micrograms of proteins : final pool in FIA

Day 0 + 135 injection by intravenous route (IV) in the tail of about 20 micrograms of proteins : final pool.
Fusion is achieved 3 days after the IV immunisation.

The sera are removed at D34, D61, D98 and D159 in order to measure the immunitary response (cf screening).

### 2.1.1 - **Screening method**

As the SPS used for the immunisations is not perfectly homogeneous, it is necessary to establish a screening test specific to this enzyme.
Two methods for the detection of the antibodies are used :

- detection method of antibodies inhibiting the SPS activity
- detection method of antibodies directed against the SPS (inhibiting or not).

**a)** Detection method of antibodies inhibiting the SPS activity This method of screening allows the detection of antibodies which interfer with the active site of the SPS or on a site close to the latter, and therefore prevent the access of substrates. In practice, 70 µl of serum or of supernatant of hybridoma culture diluted in a suitable way is mixed with 70 µl of SPS preparation (Heparin fraction). After one hour of incubation at ambient temperature, the residual SPS activity is determined by coupled enzymatic determination (Cf 1-1). The results are expressed as a percentage of inhibition as compared to the same SPS preparation treated in the same way but without antibodies.

**b)** Detection method of antibodies directed against SPS (inhibiting or not)
    This method is based on the precipitation of the antibody-SPS complex by goat anti-mouse IgG coupled to sepharose beads (GAM sepharose). In practice, 60 µl of serum or supernatant of hybridoma culture diluted in any suitable manner is added to 60 µl of SPS preparation (Heparin fraction). After 2 hours of incubation at ambient temperature, the mixture is added to 50 µl of 25 % GAM-Sepharose previously washed three times with a buffer of 50 mM HEPES, 10 mM MgCl$_2$, 1 mM EDTA, 10 % EG, 5 mM DTT, pH 7.5. The mixture is incubated over night at 4°C under strong agitation. After centrifuging for 5 minutes at 3000 rpm the residual SPS activity in the supernatant is determined by coupled enzymatic determination (Cf 1.1). The results are expressed as a percentage of precipitation (% prec.) compared to the same SPS preparation treated in the same way without antibodies.

### 2.1.2 - **Results**

10 mice were immunized according to the protocol described previously. The following table gives the results of the precipitation determinations carried out with the hetero-antisera of the 10 mice on D159. The sera are diluted to one two-hundredth.

| MOUSE | 1 | 2 | 3 | 4 | 5 | 6 | 7 | 8 | 9 | 10 |
|---|---|---|---|---|---|---|---|---|---|---|
| % PREC. | 45 | 22 | 32 | 64 | 36 | 30 | 22 | 16 | 39 | 37 |

Additional dilutions of the serum of mouse 4 give the following results :

| DILUTION | % PRECIPITATION |
|---|---|
| 1/200 | 67 |
| 1/400 | 48 |
| 1/600 | 29 |
| 1/1000 | 20 |

The spleens of mice 1 and 4 are used for the fusion.

### 2.2 - Cellular fusion

The splenocytes of the mice are fused with myeloma cells of SP2/0-Ag14 mice according to a ratio of 2/1 in the presence of 45 % polyethylene glycol 1500. The selection of the hybridomas is effected by adding hypoxanthine and azaserine to the culture medium 24 and 48 hours after fusion.
    The hybridomas are cloned and sub-cloned by the method of limited dilution.

## 2.2.1 - Results of the screening of hybrids and clones.

| HYBRIDS | |
|---|---|
| MOUSE 4 (SPA fusion) | MOUSE 1 (SPB fusion) |
| 2 postive hybrids out of 45 | 6 positive hybrids out of 52 |
| SPA2 : 38 % prec.<br><br>SPA19 : 7 % prec. | SPB3 : 17 % prec.<br><br>SPB5 : 67 % prec.<br><br>SPB8 : 53 % prec.<br><br>SPB13 : 68 % prec.<br><br>SPB25 : 13 % prec.<br><br>SPB34 : 17 % prec. |

| CLONES | |
|---|---|
| SPA FUSION | SPB FUSION |
| 2 clones retained out of 36 | 7 clones retained out of 46 |
| SPA2-2 : 85 % prec.<br><br>SPA19-7 : 8 % prec. | SPB3-2 : 19 % prec.<br><br>SPB5-1 : 76 % prec.<br><br>SPB5-2 : 71 % prec.<br><br>SPB5-3 : 45 % prec.<br><br>SPB5-4 : 24 % prec.<br><br>SPB13-1 : 79 % prec.<br><br>SPB13-2 : 53 % prec. |

| SUB-CLONES | |
|---|---|
| SPA FUSION | SPB FUSION |
| 3sub-clones retained out of 48 | 5 sub-clones retained out of 72 |
| SPA2-2-3 : 60 % prec.<br><br>SPA2-2-22 : 33 % prec.<br><br>SPA2-2-25 : 92 % prec. | SPB3-2-19 : 21 % prec.<br><br>SPB5-2-10 : 86 % prec.<br><br>SPB5-4-2 : 46 % prec.<br><br>SPB13-1-7 : 87 % prec.<br><br>SPB13-2-2 : 93 % prec. |

### 2.2.2 - **Production of anti-SPS monoclonal antibodies**

The hydridomas are injected by intra-peritoneal route into female BALB/C mice previously treated with pristane. The monoclonal antibodies are partially purified from ascital liquids thus produced by precipitation with 18 % sodium sulphate. The proteins precipitated are dissolved then dialyzed against PBS (F18).

### 2.2.3 - **Characterization of anti-SPS monoclonal antibodies**

**a)** Typing

The typing is done using an ELISA test. Anti-IgG rabbit and anti-IgM mouse antibodies (ZYMED) are fixed at the bottom of the wells of a 96-well plate. After one night at ambient temperature the unoccupied sites are saturated with a solution of 3 % bovine serum albumin in PBS. After one hour of incubation at 37°C and several washes, the various F18's are deposited in the wells. After incubation and several washes, goat or rabbit antibodies, anti-class and anti-sub class mouse immunoglobulins linked with peroxidase, are added. After one hour at 37°C, the antibodies are revealed using an $H_2O_2$/ABTS system.

All the anti-SPS monoclonal antibodies were found to be of Ig $G_1$ type.

**b)** Inhibition of SPS activity

The determination of the capacity of the antibodies to inhibit the SPS activity is carried out by the technique mentioned previously (Cf 2.1.1 a) using F18's.

| Antibody | Concentration of antibodies (micrograms/ml) | % Inhibition |
|---|---|---|
| SPA2-2-3 | 50 | 0 |
| SPA2-2-22 | 50 | 0 |
| SPA2-2-25 | 50 | 0 |
| SPB3-2-19 | 50 | 0 |
| SPB5-2-10 | 50 | 0 |
| SPB5-4-2 | 50 | 0 |
| SPB13-1-7 | 50 | 50 |
|  | 25 | 55 |
|  | 5 | 25 |
|  | 2.5 | 10 |
|  | 1 | 2.1 |
| SPB13-2-2 | 50 | 60.1 |
|  | 25 | 59.1 |
|  | 5 | 33.8 |
|  | 2.5 | 14.2 |
|  | 1 | 8.7 |

**c)** Immuno-precipitation of the SPS activity

The determination of the capacity of the antibodies to immuno-precipitate the SPS activity is carried out by the technique mentioned previously (Cf 2.1.1 b) using F18's.

| Antibody | Concentration of antibodies (micrograms/ml) | % Precipitation |
|---|---|---|
| SPA2-2-3 | 50 | 95 |
| | 25 | 92 |
| | 5 | 80 |
| | 2.5 | 40 |
| | 1 | 20 |
| SPA2-2-22 | 50 | 95.7 |
| | 25 | 95 |
| | 10 | 51 |
| | 5 | 48.2 |
| | 2.5 | 25 |
| | 1 | 10 |
| SPA2-2-25 | 50 | 91.3 |
| | 25 | 95.3 |
| | 5 | 90.4 |
| | 2.5 | 22.8 |
| | 1 | 12.5 |

| | | |
|---|---|---|
| SPB3-2-19 | 50 | 95 |
| | 25 | 95 |
| | 5 | 27.8 |
| | 2.5 | 17.8 |
| | 1 | 9.3 |
| SPB5-2-10 | 50 | 95 |
| | 25 | 95 |
| | 5 | 81.1 |
| | 2.5 | 41.4 |
| | 1 | 22.6 |
| SPB5-4-2 | 50 | 95 |
| | 25 | 95 |
| | 5 | 86.1 |
| | 2.5 | 57.2 |
| | 1 | 26.1 |
| SPB13-1-7 | 50 | 95 |
| | 25 | 95 |
| | 10 | 65.4 |
| | 5 | 48.1 |
| | 2.5 | 15 |
| | 1 | 10 |
| SPB13-2-2 | 50 | 95 |
| | 25 | 95 |
| | 5 | 71.8 |
| | 2.5 | 43.5 |

## 3 - USE OF THE MONOCLONAL ANTIBODIES FOR THE CHARACTERIZATION AND PURIFICATION OF SUCROSE PHOSPHATE SYNTHASE

### 3.1 - Characterization of Corn Sucrose Phosphate Synthase

This characterization is carried out with SPB3-2-19 and SPB13-2-2 antibodies by the technique of immuno-detection after transfer of the proteins from an electrophoresis gel under denaturing conditions (SDS-PAGE) on nitrocellulose membrane (western).

After electrophoretic separation in a 12.5 % acrylamide gel (Nature 277 (1970) 680-685), the proteins are transferred onto a 0.22 $\mu$m nitrocellulose membrane (Schleicher and Schuell). The buffer is a standard electrophoresis buffer (3.03 g/l. TRIS base, 14.4 g/l. Glycine, 0.1 % SDS, pH 8.3, 20 % methanol).

After transfer, the membrane is put in a blocking bath (0.5 % Casein in PBS). After one hour at 37°C under gentle agitation, the membrane is washed 3 to 4 times in a washing buffer (0.1 % Casein, 0.5 % Tween 20, in PBS) then incubated with a solution of 10 micrograms/ml of the monoclonal antibody to be tested. A part of the membrane is incubated in parallel with a non-immune anti-body (negative control).

After one hour of incubation at ambient temperature followed by 9 or 10 washes, the membrane is incubated in the presence of an anti-mouse antibody antibody labelled with Iodine 125 diluted in a washing buffer (50,000 cpm per cm$^2$ of membrane). After one hour of incubation at ambient temperature followed by 9 or 10 washes, the membrane is dried, then autoradiographed (X-OMAT AR KODAK film and Cronex XTRA Life DUPONT amplifying screen).

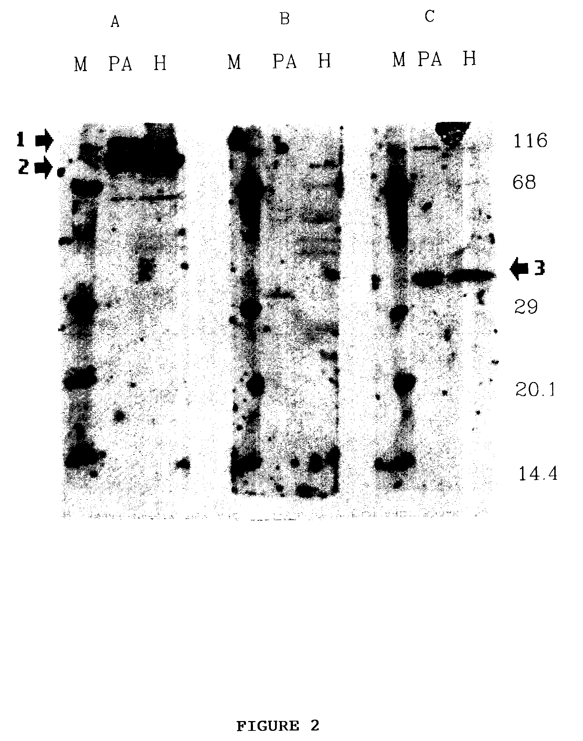

An autoradiography is shown in Figure 2. A strong signal is observed at the protein bands 120 kd, 95 kd and 35 kd which correlates with the previous results (see first part).

Key to Figure 2

A : membrane incubated in the presence of the SPB3-2-19 antibody

B : membrane incubated in the presence of an antibody not directed against SPS (negative control anti-neomycin monoclonal antibody)

C : membrane incubated in the presence of the SPB13-2-2 antibody

M : standards of molecular weight radio-marked by 125$_I$ (NEX-188 NEN) B-Galactosidase (116 kd), bovine albumin (68 kd), carbonic Anhydrase (29 kd), trypsic Inhibitor (20.1 kd), Alpha-Lactalbumin (14.4 kd), 150,000 cpm per lane

PA : proteins obtained after immunoaffinity chromatography (see below) with the SPB13-2-2 monoclonal antibody, about 40 micrograms of proteins per lane.

H : Heparin fraction, about 40 micrograms of proteins per lane.

3.2 - Purification of Sucrose Phosphate Synthase by immunoaffinity Chromatography

A methodology for the purification of corn Sucrose Phosphate Synthase on an immunoaffinity support has been perfected in order to increase the quantity of protein recovered while reducing the number of purification stages and to obtain quantities sufficient for protein sequencing.

3.2.1 - **Preparation of the immuno-adsorbent**

The F18 (see 2.2.2) corresponding to the SPB13-1-7 antibody or to the SPB13-2-2 antibody were mixed with activated CH-Sepharose, (1 mg of antibody per ml of gel). After incubation for 2 hours at ambient temperature, the sites not occupied by the antibodies are saturated with 1M ethanolamine, pH 9. The support is then washed alternately with 0.1M acetate 0.5 M NaCl pH 4 buffer and 0.1 M TRIS 0.5 M NaCl pH 8 buffer. The immunoaffinity support thus prepared is preserved at 4°C in a 50 mM HEPES, 10 mM MgCl$_2$, 1 mM EDTA, 1mM PMSF, 0.01 % sodium nitride (azide), pH 7.5 buffer.

3.2.2 - **Immunoaffinity Chromatography**

50 % PEG is added to the Heparin fraction of SPS (see 1.2.3.) is added 50 % PEG (see 1.2.1) to a final concentration of PEG of 20 %. After incubation for 30 minutes at 4°C with gentle agitation, the mixture is centrifuged at 1600 g for 30 minutes. The protein deposit is taken up in half of the initial volume with the 50 mM HEPES, 10 mM MgCl$_2$, 1 mM EDTA, 10 % ethylene glycol pH 7.5 buffer. This stage allows the previous buffer, which is incompatible with the immunoaffinity chromatography, to be eliminated, and the proteins to be concentrated. The yield of SPS activity is from 80 to 90 %.

The solution obtained is applied with a flow rate of 0.1 ml/min over 1 ml of immunoaffinity support packed in a column and on which has been fixed an antibody not directed against the SPS (activated CNBr-Sepharose, on which an anti-neomycin antibody is fixed). This first stage allows the elimination of certain contaminants which are fixed non-specifically on the chromatography support. The effluent of the non-specific column is in its turn applied to the anti-SPS immunoaffinity support (2 ml in an 11 x 20 mm column) with a flow rate of 0.1 ml/min. These two stages are carried out at laboratory temperature. The column is washed with 10 ml of load buffer and then with a washing buffer (load buffer with the addition of 0.25 M NaCl and 0.3 % Tween 20) until absorbency in ultra-violet at 280 nm is close to base level. The proteins adsorbed on the support are eluted with a solution of 50 mM triethyl-amine, pH 11. This elution is carried out at 4°C and the immunoaffinity column is reversed to obtain an optimum yield. The SDS-PAGE profile of the final preparation obtained corresponds to that obtained using the standard protocol (see 1). It must be noted that the elution method of the proteins adsorbed on the immunoaffinity support irrevesibly destroys the SPS activity but the recovery yield of the eluted SPS proteins is optimal compared to tests carried out in native elution conditions. The eluate of the immunoaffinity column is desalted with a Sephadex G25 column, against a 0.14 % Glycerol, 0.07 % 2-mercapto-ethanol, 0.04 % SDS, 0.9 mM TRIS pH 6.8 buffer (electrophoresis buffer in reducing conditions diluted 70 times). After desalification, the protein preparation is concentrated 70 times with a concentrator under vacuum and the SPS proteins are purified by SDS-PAGE (see below).

4. Partial Sequencing of SPS Polypeptides

Samples of a purified protein preparation obtained as described in Example 3.2.2. were subjected to preparative SDS-PAGE. After electrophoresis, the protein bands were visualized with KCl treatment as described by Bergman and Joernvall (Eur. Jour. Biochem. (1978) 169 : 9-12) and the bands observed at 90kd and 30kd were excised. The proteins from these gel fragments were electroeluted using an Electrophoretic Concentrator according to manufacturer's instructions (ISCO ; Lincoln, NE) in 4 mM sodium acetate, pH8. After electroelution, protein yields were quantitated by comparison to a bovine serum albumin (BSA) standard on a Coomassie Blue-stained gel. Approximately 30 $\mu$g of the 30 kd protein and 75 $\mu$g of the 90 kd protein were obtained.

The proteins were concentrated by acetone precipitation, and resuspended in 50 mM ammonium carbonate buffer, pH8. Tryptic digestion and HPLC purification were performed as described by Sturm and Chrispeels (Jour. Biol. Chem. (1987) 262 : 13392-13403). Briefly, digestion was performed by addition of trypsin (5 % of SPS protein), and incubation for two hours at 37°C. The digestion was then repeated. The proteins were concentrated by lyophilization and resuspended in 50mM sodium phosphate buffer, pH2.2. This mixture was subjected to reverse phase HPLC separation by application to a C18 column in phosphate buffer. Elution was performed using an increasing gradient of acetonitrile. Eluted material from the phosphate buffer/acetonitrile gradient was monitored at 214 nm. The fractions corresponding to peaks of absorbance at 214 nm were collected, lyophilized, resuspended in 0.1 % trifluoroacetic acid, reapplied to the C18 column (equilibrated with 0.1 % trifluoroacetic acid), and eluted using an acetonitrile gradient. Eluted material from the trifluoroacetic acid/acetonitrile gradient was monitored at 214 nm. The fractions corresponding to peaks of absorbance at 214 nm were collected, lyophilized, and subjected to standard Edman degradation protein sequencing on an automated protein sequencer (Applied Biosystems ; Foster City, CA). Sequences of 5 peptides were obtained. **Fig. 3**.

5. Isolation and Assembly of a Full-Length cDNA for SPS

5.1. RNA Isolation from Corn Leaf

Total RNA was isolated from corn leaves (see 1.2.1.) according to the method of Turpen and Griffith (Biotechniques (1986) 4 : 11-15). Briefly, 250 gm of material was homogenized in 4M guanidine thiocyanate and 2 % sarcosyl. The mixture was then centrifuged and the cleared supernatant was layered upon a 5.7M CsCl cushion and centrifuged for 5.5 hours at 50,000 RPM. The RNA pellet was dissolved in water, extracted with phenol and chloroform, and precipitated with ethanol. The resulting pellet was resuspended in water. The final yield of the RNA isolation was quantitated by UV spectrophotometry.

5.2. Poly(A) RNA Isolation

A saturated suspension of cellulose powder/water was added to the RNA/water mixture, at 10 % of the total volume, to remove residual polysaccharides. After centrifugation, the supernatant, containing the RNA, was applied to an oligo(dT)-cellulose column as described by Maniatis et al. (Molecular Cloning : A Laboratory Manual, (1982) Cold Spring Harbor, New York). The fraction containing the poly(A)+ RNA was then reapplied to the column. The eluted fraction containing the poly(A)+ RNA was extracted with phenol, and the RNA was precipitated with ethanol. Analysis by gel electrophoresis showed complete absence of ribosomal RNA.

5.3. Construction of Total Corn Leaf Library

cDNA synthesis was performed according to manufacturer's instructions (RiboClone cDNA Synthesis System by Promega, Madison, WI), using five $\mu$g of poly(A)+ RNA as template, except that M-MLV reverse transcriptase (BRL ; Bethesda, MD) was substituted for AMV reverse transcriptase. EcoRI linkers were added to the blunt-ended cDNA, and the resulting fragments were cloned into an expression vector (LambdaZAP, Stratagene ; La Jolla, CA) according to manufacturer's instructions. The resulting library contained approximately 1.5 x 10$^6$ transformants.

5.4. PCR Generation of a Partial SPS cDNA Probe

Using the sequence information from the peptides of Example 4 and the polymerase chain reaction (PCR), a 1200 bp SPS cDNA fragment was generated. Total corn leaf cDNA (5.3.) was used as a template, and degenerate oligonucleotides, designed from two peptide sequences of the 30kd and 90kd SPS polypeptides, were used as primers. These primer sets were designated as CD3 and CD4. **Fig. 4**. PCR was carried out, according to manufacturer's instructions (GeneAmp DNA Amplification Reagent Kit and DNA Thermal Cycler of Perkin Elmer Cetus ; Norwalk, CT) except that the reaction was carried out for 30 cycles, and the annealing steps were programmed to be 50°C for 1 minute. The PCR reactions were analyzed by agarose gel electrophoresis. Use of the correct set of primers, which was CD3, resulted in a 1200 bp band being generated by the PCR reaction. PCR using the other set of primers, CD4, gave no specific signals. **Fig. 5**. Southern analysis confirmed that the PCR band was not an artifact, as shown in **Fig. 5**. The probe 4K5 was used in that the corresponding sequence of the probe was predicted to be within the 1200bp fragment if the fragment corresponded to the SPS sequence. The probe hybridized to the 1200 bp band generated by PCR using the primer set CD3 but not to PCR products generated by the primer set CD4 **Fig. 5**.

## 5.5 Isolation of SPS Bacteriophage Lambda cDNA Clones

The 1200 bp PCR-generated fragment was labeled with $^{32}$P (as per the Random Primed DNA Labeling Kit, Boehringer Mannheim, Indianapolis, IN) and used as a probe to screen approximately 250,000 plaques of the cDNA library (5.3.). The inserts of the positive clones were analyzed by restriction analysis with EcoRI, and the clones with the longest inserts, SPS#3 and SPS#18, were selected for further analysis. **Fig. 6.** A 0.4 kb HindIII/EcoRI fragment from the 5' end of SPS#3 was isolated, then labeled with $^{32}$P by random priming (Random Primed DNA Labeling Kit) and used as a probe to re-screen the library. Another clone, designated SPS#61, which extends further upstream than SPS#3, was isolated. **Fig. 6**. DNA sequencing indicated that the 5' end of the SPS reading frame was not reached.

To isolate cDNA clones that included more of the 5' region than SPS#3 or SPS#61, a new cDNA library was prepared, as per Example 5.3., (RiboClone cDNA Synthesis System by Promega ; Madison, WI) using M-MLV reverse transcriptase instead of AMV reverse transcriptase. However, instead of using oligo (dT) as a primer, a synthetic 17 bp primer, 23B, derived from the 5' sequence of the SPS#61 clone, was used (**Fig. 6**). This resulted in cDNAs that only contain regions upstream of the the SPS#61 5' region. The library was screened with the $^{32}$P-labeled EcoRI insert from SPS#61, and 16 positive clones were obtained. The clones with the longest inserts, SPS#77 and SPS#90, were selected for further analysis. DNA sequencing of SPS#77 and SPS#90 showed that the region of overlap (greater than 100 bp) with SPS#61 was identical in all clones, and that both extend further upstream into the 5' region. **Fig. 6**

PCR was carried out using single-stranded cDNA (from a reverse transcriptase reaction corn leaf RNA (5.2.) primed with oligo (dT) T) as described above) as template and primers selected from the SPS#90 and SPS#3 sequences, confirmed that SPS#90 and SPS#3 originate from the same mRNA transcript. The fragment resulting from this PCR reaction was 750 bp in length, consistent with the the size predicted from the DNA sequence. The 750 bp fragment was subcloned into a Bluescript-derived vector as a SalI/HindIII fragment. Four of the resulting subclones were partially sequenced, and the sequence obtained matched the existing DNA sequence.

## 5.6. Assembly of the SPS Reading Frame.

Both DNA strands of #90, #61, and #3 were sequenced, using the method of Sanger et al. (PNAS (1977) 74 : 5463-5467). All three sequences can be combined to one contiguous sequence of 3509 bp, **Fig. 7**. Primer extension experiments using corn leaf poly(A) RNA and an antisense primer showed that the 5' end of our DNA sequence represents sequences form the actual 5' end of the SPS in RNA. In the SPS reading frame, as defined by the five peptide sequences (**Fig. 3**), the first methionine codons are located at bp 112 and bp 250. **Fig. 7.** The codon at bp 112 is similar to the consensus eukaryotic translational start site (Kozak, Cell (1986) 44 : 283-292) and is located 54 bp downstream of a TAG stop codon (bp 58). It is proposed that this codon represents the translational start of the SPS polypeptide in vivo. After a 1068 codon reading frame, translation is stopped by TGA. The following 193 bp contain the 3' untranslated region including a poly(A) addition signal, AAATAAA.

The full-length SPS coding region may be assembled by combining the 529 bp BamHI/HindIII fragment of SPS#90, the 705 bp HindIII fragment of SPS#61 and the 2162 bp HindIII/EcoRI fragment from SPS#3 (see **Fig. 6**).

## 6. Detection of SPS Polypeptides by Specific Antisera

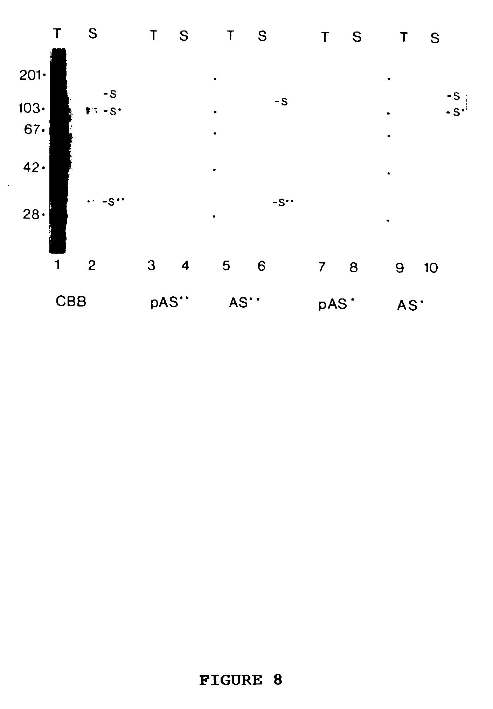

Samples of purified protein preparations obtained by the method described in 3.2.2. were subjected to SDS-PAGE electrophoresis. The proteins in the gel were fixed and stained. The bands corresponding to the 90kd and 30kd polypeptides were excised. With this material polyclonal antisera were raised in rabbits by conventional procedures. Western analysis (as described by Oberfelder, Focus (1989) 11(1) : 1-5) showed that the antibodies isolated from the rabbit immunized with SPS 30 recognized the bands corresponding to the SPS 30 and SPS 120 peptides on a SDS PAGE gel, and that the antibodies isolated from the rabbit immunized with SPS 90 recognized the bands corresponding to the SPS 90 and SPS 120 polypeptides **Fig.8.**

## 6.2. Immunological Localization of SPS in the Corn Plant

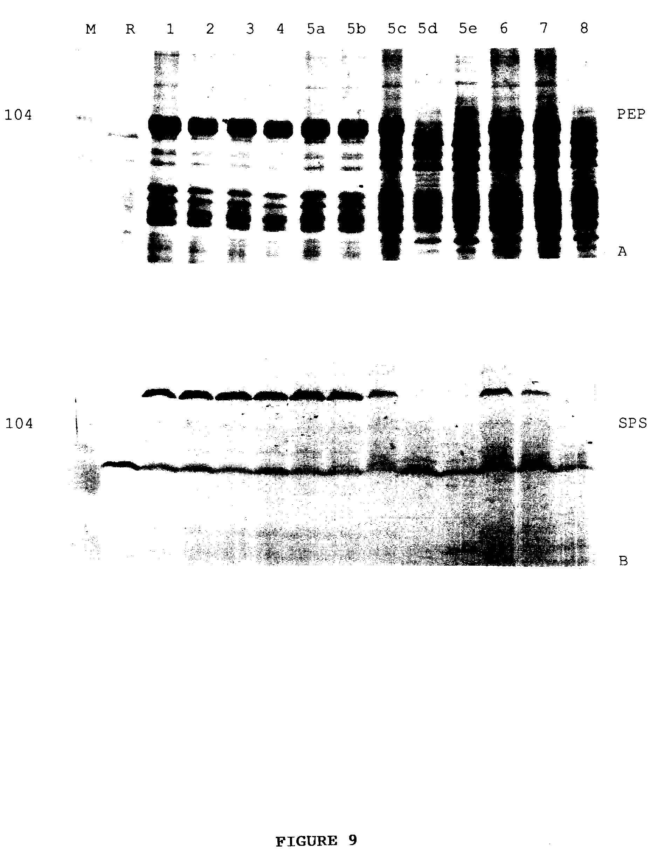

Total proteins were extracted from leaves of a 30 day-old corn plant, harvested at 11 : 00 AM, by boiling in SDS buffer. The protein extracts were loaded on duplicate SDS-PAGE gels. One gel was stained with Coomassie Blue, while the other was subjected to Western analysis, using a mixture of SPS30 and SPS90 antisera as probe. **Fig. 9**. The prominent bands appearing on the Coomassie Blue-stained gel were identified as phosphoenolpyruvate carboxylase (PEPcase), an enzyme involved in C4 photosynthesis. The Western blot showed the presence of the SPS band. The SPS protein pattern was very similar to the PEPcase protein pattern : not present in roots, and not present in the section of leaf closest to the the stem, or in very young leaves. This pattern corresponds with expression associated with photosynthesis, and is the pattern expected for SPS.

## 7. Construction of Expression Constructs Plasmids

### 7.1. Construction of the full-length SPS reading frame

Clone SPS#90 is digested with HindIII and ligated with the 705 bp HindIII fragment from clone SPS#61 to create a plasmid containing the 5' end of the SPS coding region. The resulting plasmid is digested with BamHI and partially digested with HindIII, resulting in a 1340 bp BamHI/HindIII fragment containing the 5' end of the coding region. The 3' end of the SPS coding region is obtained by digestion of SPS#3 with EcoRI and partial digestion with HindIII, resulting in a 2162 bp HindIII/EcoRI fragment. This 2162 bp HindIII/EcoRI fragment, carrying the 3' end, is ligated with the 1340 BamHI/EcoRI fragment carrying the 5' end into a BamHI/EcoRI-digested pUC-derivative plasmid Bluescript, to create a plasmid carrying the entire 3403 bp SPS coding region and 3' untranslated transcription termination region.

### 7.2 Expression of SPS in E. coli

When cloning the 3403 bp BamHI/EcoRI SPS fragment into the plasmid Bluescript SK (Stratagene, La Jolla, CA), a translational fusion between the plasmid coded lacZ sequence and the SPS reading frame is created. The resulting fusion protein contains 30 N-terminal amino acids from the betagalactosidase and the complete SPS polypeptide. The fusion protein was expressed in E. coli under the Bluescribe plasmid lacZ promoter. Preparation of total protein followed by Western analysis using anti SPS antisera (6.1.) shows a band comigrating with native plant SPS. For SPS activity test the E. coli cells containing the SPS expression construct as described were opened with Lysozyme and sonication. Soluble protein was desalted by a Sephadex G-25 column. This protein extract was assayed for SPS activity analogous to (1.1.a.) except the reagent anthrone was used instead of resovcinol (E.U. Handel, Analytical Biochemistry, (1968) 22 : 280-283). This test shows that the SPS protein, expressed from the cDNA in E. coli does have SPS enzyme activity. By comparison to native plant enzyme it seems to have the same specific activity.

### 7.3. Construction of the Tobacco Small Subunit (SSU) Promoter-Transcriptional Fusions

The SPS coding region can be conveniently cloned as a BamHI/EcoRI (bp 106 - bp 3506) fragment 3' of a tobacco small subunit promoter.

A SSU promoter for expression of the SPS coding region, may be prepared as follows. The SSU promoter region from **PCGN627**(described below) is opened by KpnI and the 3' overhang removed. After EcoRI digestion, the 3403 bp BamHI (filled in) EcoRI SPS cDNA fragment (see, Example 7.1.) can be inserted.

After the SPS coding region is ligated into the SSU promoter, the SSU/SPS region may be ligated into a binary vector and integrated into a plant genome via Agrobacterium tumefaciens mediated transformation (The SPS region carries its own transcription termination region in the cDNA sequence.)

### pCGN627

The 3.4 kb EcoRI fragment of TSSU3-8 (O'Neal et al., Nucleic Acids Res. (1987) 15 : 9661-8677), containing the small subunit promoter region, is cloned into the EcoRI site of **M13mp18** (Yanisch-Perron et al, Gene (1985) 53 : 103-119) to yield an M13 clone **8B**. Single-stranded DNA is used as a template to extend oligonucleotide primer "Probe 1" (O'Neal et al., Nucleic Acids Research (1987) 15 : 8661-8677) using the Klenow fragment of DNA polymerase I. Extension products are treated with mung bean nuclease and then digested with HindIII to yield a 1450 bp fragment containing the SSU promoter region. The fragment is cloned into HindIII-SmaI-digested **pUC18** (Yanisch-Perron et al., Gene (1985) 53 : 103-119) to yield **pCGN625**.

**pCGN625** is digested with HindIII, the ends blunted with Klenow, and the digested plasmid re-digested with EcoRI. The EcoRI/blunted-HindIII fragment containing the SSU promoter region is ligated with SmaI/EcoRI-digested **pUC18** to yield **pCGN627**.

### 7.4. Construction of a CaMV Promoter - SPS Transcriptional Fusion

The 35S promoter DNA fragment from cauliflower mosaic virus can be fused to the SPS DNA as follows.

The plasmid pCGN639 can be opened by BamHI and EcoRI and the 3403 bp BamHI/EcoRI SPS cDNA fragment as described in Example 7.1 can be cloned into this plasmid. The hybrid gene can be removed from this plasmid as a 4.35 kb XbaI EcoRI fragment and ligated into a binary vector (K.E. McBride and K.R. Summerfelt, Plant Mol. Bio. (1990) 14 : 269-276) and integrated into a plant genome via Agrobacterium tumefaciens mediated transformation.

### 7.4.1. Construction of pCGN639

pCGN164 is digested with EcoRV and BamHI to release a EcoRV-BamHI fragment which contained a portion of the 35S promoter (bp 7340-7433). pCGN638 is digested with HindIII and EcoRV to release a HindIII-EcoRV fragment containing a different portion of the 35S promoter (bp 6493-7340). These two fragments are ligated into pCGN986 which has been digested with HindIII and BamHI to remove the HindIII-BamHI fragment containing the 35S-promoter ; this ligation produces pCGN639, which contains the backbone and tml-3' region from pCGN986 and the two 35S promoter fragments from pCGN164 and pCGN638.

### 7.4.2. Construction of pCGN164

The AluI fragment of CaMV (bp 7144-7735) (Gardner et al., Nucl.Acids Res. (1981) 9 : 2871-2888) is obtained by digestion with AluI and cloned into the HincII site of M13mp7 (Vieira and Messing, Gene (1982) 19 : 259-268) to create C614. An EcoRI digest of C614 produces the EcoRI fragment from C614 containing the 35S promoter which is cloned into the EcoRI site of pUC8 (Vieira and Messing, supra) to produce pCGN146. To trim the promoter region, the BglII site (bp 7670) is treated with BglII and Bal31 and subsequently a BglII linker is attached to the Bal31 treated DNA to produce pCGN147. pCGN147 is digested with EcoRI/HphI and the resulting EcoRI-HphI fragment containing the 35S promoter is ligated into EcoRI-SmaI digested M13mp8 (Vieira and Messing, supra) to create pCGN164.

### 7.4.3. Construction of pCGN638

Digestion of CaMV10 (Gardner, et al., Nucl. Acids Res. (1981) 9 : 2871-2888) with BglII produces a BglII fragment containing a 35S promoter region (bp 6493-7670) which is ligated into the BamHI site of pUC19 (Norrander et al., Gene (1983) 26 : 101-106) to create pCGN638.

### 7.4.4. Construction of pCGN986

pCGN986 contains a cauliflower mosaic virus 35S (CaMV35) promoter and a T-DNA tml-3' region with multiple restriction sites between them. pCGN986 is derived from another cassette, pCGN206, containing a CaMV35S promoter and a different 3' region, the CaMV region VI 3'-end and pCGN971E, a tml 3' region.

pCGN148a containing a promoter region, selectable marker (kanamycin with 2 ATG's) and 3' region, is prepared by digesting pCGN528 with BglII and inserting the BamHI-BglII promoter fragment from pCGN147 (see above). This fragment is cloned into the BglII site of pCGN528 so that the BglII site is proximal to the kanamycin gene of pCGN528.

The shuttle vector used for this construct pCGN528, is made as follows : pCGN525 was made by digesting a plasmid containing Tn5, which harbors a kanamycin gene (Jorgensen et al., Mol. Gen. Genet. (1979) 177 : 65), with HindIII-BamHI and inserting the HindIII-BamHI fragment containing the kanamycin resistance gene into the HindIII-BamHI sites in the tetracycline gene of pACYC184 (Chang and Cohen, J. Bacteriol. (1978) 134 : 1141-1156). pCGN526 was made by inserting the BamHI fragment 19 of pTiA6 (Thomashow et al., Cell (1980) 19 : 729-739) modified with XhoI linkers inserted into the SmaI site, into the BamHI site of pCGN525. pCGN528 was obtained by deleting the small XhoI and religating.

pCGN149a is made by cloning the BamHI kanamycin gene fragment from pMB9KanXXI into the BamHI site of pCGN148a. pMB9KanXXI is a pUC4K variant (Vieira and Messing, Gene (1982) 19 : 259-268) which has the XhoI site missing but contains a function kanamycin gene from Tn903 to allow for efficient selection in Agrobacterium.

pCGN149a is digested with HindIII and BamHI and ligated which pUC8 (Vieira and Messing, supra) digested with HindIII and BamHI to produce pCGN169. This removes the Tn903 kanamycin marker. pCGN565 and pCGN169 are both digested with HindIII and PstI and ligated to form pCGN203, a plasmid containing the CaMV 35S promoter and part of the 5'-end of the Tn5 kanamycin gene (up to the PstI site, (Jorgensen et al., Mol. Gen. Genet. (1979) 177 : 65). pCGN565 is a cloning vector based on pUC8-Cm (K. Buckley, Ph.D. Thesis, UC San Diego 1985), but containing the polylinker from pUC18 (Yanisch-Perron et al., Gene (1985) 53 : 103-119).

A 3' regulatory region is added to pCGN203 from pCGN204 (an EcoRI fragment of CaMV (bp 408-6105) containing the region VI 3' cloned into pUC18 (Gardner et al., Nucl. Acids Res. (1981) 9 : 2871-2888) by digestion with HindIII and PstI and ligation. The resulting cassette, pCGN206, is the basis for the construction of pCGN986.

The pTiA6 T-DNA tml 3'-sequences are subcloned from the Bam19 T-DNA fragment (Thomashow et al., Cell (1980) 19 : 729-739) as a BamHI-EcoRI fragment (nucleotides 9062 to 12, 823, numbering as in (Barker et al., Plant Mo. Biol. (1983) 2 : 335-350) and combined with the **pACYC184** (Chang and Cohen, J. Bacteriol. (1978) 134 : 1141-1156) origin of replication as an EcoRI-HindII fragment and a gentamycin resistance marker (from plasmid **pLB41**), (D. Figurski) as a BamHI-HindII fragment to produce **pCGN417**.

The unique SmaI site of **pCGN417** (nucleotide 11,207 of the Bam19 fragment) is changed to a SacI site using linkers and the BamHI-SacI fragment is subcloned into **pCGN565** to give **pCGN971**. The BamHI site of **pCGN971** is changed to an EcoRI site using linkers to yield **pCGN971E**. The resulting EcoRI-SacI fragment of **pCGN971E**, containing the tml 3' regulatory sequence is joined to **pCGN206** by digestion with EcoRI and SacI to give **pCGN975**. The small part of the Tn5 kanamycin resistance gene is deleted from the 3'-end of the CaMV 35S promoter by digestion with SalI and BglII, blunting the ends and ligating with SalI linkers. The final expression cassette **pCGN986** contains the CaMV 35S promoter followed by two SalI sites, an XbaI site, BamHI, SmaI, KpnI and the tml 3' region (nucleotides 11207-9023 of the T-DNA).

Here under are indication schemes of the constructs.

pCGN627

TSSU3-8

M13mp18

8B

primer

pUC18

pCGN265

pUC18

pCGN2657


pCGN639

AluI frag. of CaMV                    CaMV10

M13mp7                                      pCU19

C614                              pCGN638

pUC8

pCGN146

pCGN147                          pCGN986

M13mp8

pCGN164                          pCGN639

24

<u>pCGN986</u>

pCGN528

pCGN147 (see 639 desc.)

pCGN148a

pUB9KanXXI

pCGN149a

pUC8-Cm

pUC18                              pUC8

pCGN565                    pCGN169

pCGN203

CaMV

pCGN204

pUC18

pCGN206

pCGN975          pCGN986

pCGN971E

pCGN971

pCGN565

pCGN417

pTiA6 + pACYC184 + pLB41


<u>Detailed description of the figures</u>

Figure 1
Figure 2
Figure 3 : shows peptide sequences derived from SPS protein. All peptides are typed N -> C terminal.

Figure 4 : shows oligonucleotides used for the PCR reactions CD3 and CD4 in relation to the peptides (antisense sequences are presented upside down). Arrows point to the direction the oligonucleotides will prime the polymerase.

Figure 5A : shows agarose gel electrophoresis of CD3 and CD4 PCR reactions. The sizes are given in kb.

Figure 5B : shows autoradiograph of Southern blot of CD3 and CD4 PCF reactions probed with oligonucleotides 4k5.

Figure 6 : shows schematic diagrams representing SPS cDNA and selected clones. The upper bar represents the entire 3509 bp combined map. Translation stop and start are indicated.

Figure 7 : shows the assembled SPS cDNA sequence. The sequences of clones SPS 90, SPS 61 and SPS 3 were fused at the points indicated in Fig. 2. The SPS reading frame is translated. All SPS protein derived peptide sequences are indicated.

Figure 8 : shows Westerne blots showing characteristics of rabbit SPS 90 and SPS 30 antisera. pAS** = preimmune serum, SPS 30 rabbit ; AS** = immune serum anti SPS 90. Molecular weight markers at left, where indicated. S = SPS 120 kd polypeptide ; S* = SPS 90 kd polypeptide ; S** = SPS 30 kd polypeptide.

Figure 9A : shows a Comassie Blue-stained gel of total protein isolated from a 30 day old corn plant. M = size marker ; R = roots ; 1-8 = leaf numbers counting from the bottom of the plant. Leaf 5 has been cut into 5 segments from the leaf tip (5a) to the end of the sheath (5c). PEP = phosphoenolpyruvate carboxylase.

Figure 9B : shows the results of Western blot analysis using a mixture of antiSPS 30 and antiSPS 90 antisera against total plant protein isolated from a 30 day old corn plant. The signal corresponding to SPS appears at 120-140 kd.

## Claims

1. Protein derivatives from the nature SPS proteins substantially free of other proteins and being capable of catalyzing the formation of sucrose phosphate, modified by genetic engeneering and having the SPS activity.

2. Protein derivatives of Claim 1 wherein the nature SPS protein is a sucrose phosphate synthase (SPS) having fructose-6-phosphate and UDP glucose substrate.

3. Protein derivatives of Claim 1 wherein the nature SPS derivative is a corn SPS having a molecular weight from about 100 to 130 Kd and a specific activity of greater then 0,05U.

4. Protein derivatives of Claim 3 wherein the nature SPS derivative has a specific activity of about 25U.

5. Protein derivatives of Claim 1 wherein the nature SPS derivatives has an amino sequence according to Fig. 7.

6. An estrachromosomal DNA sequence comprising sequence encoding a sucrose phosphate synthase (SPS).

7. The sequence of Claim 6 obtainable from corn.

8. The sequence of Claim 6 comprising cDNA.

9. The sequence of Claim 6 comprising genomic DNA.

10. The sequence of Claim 6 joined to a second DNA sequence which is different from sequences found joined together in nature.

FIGURE 1

FIGURE 2

SPS 90 kd PEPTIDES

A8   ThrTrpIleLys

B4   TyrValValGluLeuAlaArg

B11  SerMetProProIleTrpAlaGluValMetArg


SPS 30 kd PEPTIDES


4K   LeuArgProAspGlnAspTyrLeuMetHisIleSerHisArg

12N  TrpSerHisAspGlyAlaArg


FIGURE 3

B11
SerMetProProIleTrpAlaGluValMetArg
TCAATGCCACCAATATGGGCAGAAGTAATGCGA
AGC   C   C   C   G   C   A C   G
      G   G   T   G   G       G
      T   T       T   T        T

4K
LeuArgProAspGlnAspTyrLeuMetHisIleSerHisArg
TTAAGACCAGACCAAGACTACTTAATGCACATAAGACACAGA
C GC C   C   T G   T   TC C   T   CTCC   TC C   G
       C   G   G           G          T   G     T
       T   T       T             T

CD3 B11C  ATGCCICCIATATGGGCIGA ---->          <---- CTAGTCCTAATAGAITACGT   4K3
               C   C   C                              G   T   G   G   C
                   T

CD4 B11B <-- TACGGIGGITAAACCCGICT       GACCAAGACTACCTIATGCA ----->   4K1S
              C   C   G                  T   G   T   T   C
                  T

B11A  ACCCGICTCCAITACGC              TACGTATAATCAGTAGC   4K2
        C   T   C   T                 G   G   GT
                                          T

AAIGCIGGICTAGT   4K5
 G CT C   C   G

FIGURE 4

CD3   CD4    M                    CD3  CD4    M

1.4
1.1

A                                                          B

FIGURE 5

**FIGURE 6**

SPS cDNA sequence

```
     EcoRI
     |
   1 GAATTCCGGCGTGGGCGCTGGGCTAGTGCTCCCGCAGCGAGCGATCTGAGAGAACGGTAGAGTTCCGGC   69
     2


                                                  BamHI
                                                  |
  70 CGGGCGCGCGGGAGAGGAGGAGGGTCGGGCGGGGAGGATCCGATGGCCGGGAACGAGTGGATCAATGGG   138
                                                   METAlaGlyAsnGluTrpIleAsnGly
                                              106


     KpnI
     |
 139 TACCTGGAGGCGATCCTCGACAGCCACACCTCGTCGCGGGGTGCCGGCGGCGGCGGCGGCGGGGGGGGAC   207
     TyrLeuGluAlaIleLeuAspSerHisThrSerSerArgGlyAlaGlyGlyGlyGlyGlyGlyGlyAsp
     142


 208 CCCAGGTCGCCGACGAAGGCGGCGAGCCCCCGCGGCGCGCACATGAACTTCAACCCCTCGCACTACTTC   276
     ProArgSerProThrLysAlaAlaSerProArgGlyAlaHisMETAsnPheAsnProSerHisTyrPhe


                         SalI
                         |
 277 GTCGAGGAGGTGGTCAAGGGCGTCGACGAGAGCGACCTCCACCGGACGTGGATCAAGGTCGTCGCCACC   345
     ValGluGluValValLysGlyValAspGluSerAspLeuHisArgThrTrpIleLysValValAlaThr
                         299                          A8


                     XhoI
                     |
 346 CGCAACGCCCGCGAGCGCAGCACCAGGCTCGAGAACATGTGCTGGCGGATCTGGCACCTCGCGCGCAAG   414
     ArgAsnAlaArgGluArgSerThrArgLeuGluAsnMETCysTrpArgIleTrpHisLeuAlaArgLys
                                            374


 415 AAGAAGCAGCTGGAGCTGGAGGGCATCCAGAGAATCTCGGCAAGAAGGAAGGAACAGGAGCAGGTGCGT   483
     LysLysGlnLeuGluLeuGluGlyIleGlnArgIleSerAlaArgArgLysGluGlnGluGlnValArg


 484 CGTGAGGCGACGGAGGACCTGGCCGAGGATCTGTCAGAAGGCGAGAAGGGAGACACCATCGGCGAGCTT   552
     ArgGluAlaThrGluAspLeuAlaGluAspLeuSerGluGlyGluLysGlyAspThrIleGlyGluLeu


 553 GCGCCGGTTGAGACGACCAAGAAGAAGTTCCAGAGGAACTTCTCTGACCTTACCGTCTGGTCTGACGAC   621
     AlaProValGluThrThrLysLysLysPheGlnArgAsnPheSerAspLeuThrValTrpSerAspAsp


                     HindIII
                     |
 622 AATAAGGAGAAGAAGCTTTACATTGTGCTCATCAGCGTGCATGGTCTTGTTCGTGGAGAAAACATGGAA   690
     AsnLysGluLysLysLeuTyrIleValLeuIleSerValHisGlyLeuValArgGlyGluAsnMETGlu
                                            635


 691 CTAGGTCGTGATTCTGATACAGGTGGCCAGGTGAAATATGTGGTCGAACTTGCAAGAGCGATGTCAATG   759
     LeuGlyArgAspSerAspThrGlyGlyGlnValLysTyrValValGluLeuAlaArgAlaMETSerMET
                                                B4


 760 ATGCCTGGAGTGTACAGGGTGGACCTCTTCACTCGTCAAGTGTCATCTCCTGACGTGGACTGGAGCTAC   828
     METProGlyValTyrArgValAspLeuPheThrArgGlnValSerSerProAspValAspTrpSerTyr
```

FIGURE 7  1/4

```
 829 GGTGAGCCAACCGAGATGTTATGCGCCGGTTCCAATGATGGAGAGGGGATGGGTGAGAGTGGCGGAGCC  897
     GlyGluProThrGluMETLeuCysAlaGlySerAsnAspGlyGluGlyMETGlyGluSerGlyGlyAla

 898 TACATTGTGCGCATACCGTGTGGGCCGCGGGATAAATACCTCAAGAAGGAAGCGTTGTGGCCTTACCTC  966
     TyrIleValArgIleProCysGlyProArgAspLysTyrLeuLysLysGluAlaLeuTrpProTyrLeu

 967 CAAGAGTTTGTCGATGGAGCCCTTGCGCATATCCTGAACATGTCCAAGGCTCTGGGAGAGCAGGTTGGA 1035
     GlnGluPheValAspGlyAlaLeuAlaHisIleLeuAsnMETSerLysAlaLeuGlyGluGlnValGly

1036 AATGGGAGGCCAGTACTGCCTTACGTGATACATGGGCACTATGCCGATGCTGGAGATGTTGCTGCTCTC 1104
     AsnGlyArgProValLeuProTyrValIleHisGlyHisTyrAlaAspAlaGlyAspValAlaAlaLeu

1105 CTTTCTGGTGCGCTGAATGTGCCAATGGTGCTCACTGGCCACTCACTTGGGAGGAACAAGCTGGAACAA 1173
     LeuSerGlyAlaLeuAsnValProMETValLeuThrGlyHisSerLeuGlyArgAsnLysLeuGluGln

1174 CTGCTGAAGCAAGGGCGCATGTCCAAGGAGGAGATCGATTCGACATACAAGATCATGAGGCGTATCGAG 1242
     LeuLeuLysGlnGlyArgMETSerLysGluGluIleAspSerThrTyrLysIleMETArgArgIleGlu

1243 GGTGAGGAGCTGGCCCTGGATGCGTCAGAGCTTGTAATCACGAGCACAAGGCAGGAGATTGATGAGCAG 1311
     GlyGluGluLeuAlaLeuAspAlaSerGluLeuValIleThrSerThrArgGlnGluIleAspGluGln
```

```
                                        HindIII
                                           |
1312 TGGGGATTGTACGATGGATTTGATGTCAAGCTTGAGAAAGTGCTGAGGGCACGGGCGAGGCGCGGGGTT 1380
     TrpGlyLeuTyrAspGlyPheAspValLysLeuGluLysValLeuArgAlaArgAlaArgArgGlyVal
                                          1340
```

```
            NcoI
             |
1381 AGCTGCCATGGTCGTTACATGCCTAGGATGGTGGTGATTCCTCCGGGAATGGATTTCAGCAATGTTGTA 1449
     SerCysHisGlyArgTyrMETProArgMETValValIleProProGlyMETAspPheSerAsnValVal
            1387
```

```
1450 GTTCATGAAGACATTGATGGGGATGGTGACGTCAAAGATGATATCGTTGGTTTGGAGGGTGCCTCACCC 1518
     ValHisGluAspIleAspGlyAspGlyAspValLysAspAspIleValGlyLeuGluGlyAlaSerPro

1519 AAGTCAATGCCCCCAATTTGGGCCGAAGTGATGCGGTTCCTGACCAACCCTCACAAGCCGATGATCCTG 1587
     LysSerMETProProIleTrpAlaGluValMETArgPheLeuThrAsnProHisLysProMETIleLeu
                                   B11
```

```
1588 GCGTTATCAAGACCAGACCCGAAGAAGAACATCACTACCCTCGTCAAAGCGTTTGGAGAGTGTCGTCCA 1656
     AlaLeuSerArgProAspProLysLysAsnIleThrThrLeuValLysAlaPheGlyGluCysArgPro

1657 CTCAGGGAACTTGCAAACCTTACTCTGATCATGGGTAACAGAGATGACATCGACGACATGTCTGCTGGC 1725
     LeuArgGluLeuAlaAsnLeuThrLeuIleMETGlyAsnArgAspAspIleAspAspMETSerAlaGly

1726 AATGCCAGTGTCCTCACCACAGTTCTGAAGCTGATTGACAAGTATGATCTGTACGGAAGCGTGGCGTTC 1794
     AsnAlaSerValLeuThrThrValLeuLysLeuIleAspLysTyrAspLeuTyrGlySerValAlaPhe
```

```
                              BglII
                                |
1795 CCTAAGCATCACAATCAGGCTGACGTCCCGGAGATCTATCGCCTCGCGGCCAAAATGAAGGGCGTCTTC 1863
     ProLysHisHisAsnGlnAlaAspValProGluIleTyrArgLeuAlaAlaLysMETLysGlyValPhe
                                           1827
```

```
1864 ATCAACCCTGCTCTCGTTGAGCCGTTTGGTCTCACCCTGATCGAGGCTGCGGCACACGGACTCCCGATA 1932
     IleAsnProAlaLeuValGluProPheGlyLeuThrLeuIleGluAlaAlaAlaHisGlyLeuProIle
```

FIGURE 7  2/4

```
                                  SalI
                                   |
1933 GTCGCTACCAAGAATGGTGGTCCGGTCGACATTACAAATGCATTAAACAACGGACTGCTCGTTGACCCA 2001
     ValAlaThrLysAsnGlyGlyProValAspIleThrAsnAlaLeuAsnAsnGlyLeuLeuValAspPro
                                  1958

                                       HindIII
                                         |
2002 CACGACCAGAACGCCATCGCTGATGCACTGCTGAAGCTTGTGGCAGACAAGAACCTGTGGCAGGAATGC 2070
     HisAspGlnAsnAlaIleAlaAspAlaLeuLeuLysLeuValAlaAspLysAsnLeuTrpGlnGluCys
                                       2036

2071 CGGAGAAACGGGCTGCGCAACATCCACCTCTACTCATGGCCGGAGCACTGCCGCACTTACCTCACCAGG 2139
     ArgArgAsnGlyLeuArgAsnIleHisLeuTyrSerTrpProGluHisCysArgThrTyrLeuThrArg

2140 GTGGCCGGGTGCCGGTTAAGGAACCCGAGGTGGCTGAAGGACACACCAGCAGATGCCGGAGCCGATGAG 2208
     ValAlaGlyCysArgLeuArgAsnProArgTrpLeuLysAspThrProAlaAspAlaGlyAlaAspGlu

                              NcoI
                               |
2209 GAGGAGTTCCTGGAGGATTCCATGGACGCTCAGGACCTGTCACTCCGTCTGTCCATCGACGGTGAGAAG 2277
     GluGluPheLeuGluAspSerMETAspAlaGlnAspLeuSerLeuArgLeuSerIleAspGlyGluLys
                              2229

2278 AGCTCGCTGAACACTAACGATCCACTGTGGTTCGACCCCCAGGATCAAGTGCAGAAGATCATGAACAAC 2346
     SerSerLeuAsnThrAsnAspProLeuTrpPheAspProGlnAspGlnValGlnLysIleMETAsnAsn

2347 ATCAAGCAGTCGTCAGCGCTTCCTCCGTCCATGTCCTCAGTCGCAGCCGAGGGCACAGGCAGCACCATG 2415
     IleLysGlnSerSerAlaLeuProProSerMETSerSerValAlaAlaGluGlyThrGlySerThrMET

2416 AACAAATACCCACTCCTGCGCCGGCGCCGGCGCTTGTTCGTCATAGCTGTGGACTGCTACCAGGACGAT 2484
     AsnLysTyrProLeuLeuArgArgArgArgArgLeuPheValIleAlaValAspCysTyrGlnAspAsp

                              PstI
                               |
2485 GGCCGTGCTAGCAAGAAGATGCTGCAGGTGATCCAGGAAGTTTTCAGAGCAGTCCGATCGGACTCCCAG 2553
     GlyArgAlaSerLysLysMETLeuGlnValIleGlnGluValPheArgAlaValArgSerAspSerGln
                              2511

          BglII                    SalI                              PstI
            |                       |                                 |
2554 ATGTTCAAGATCTCAGGGGTTCACGCTGTCGACTGCCATGCCGTTGTCCGAGACACTCCAGCTTCTGCAG 2622
     METPheLysIleSerGlyPheThrLeuSerThrAlaMETProLeuSerGluThrLeuGlnLeuLeuGln
            2562                    2581                              2622

2623 CTCGGCAAGATCCCAGCGACCGACTTCGACGCCCTCATCTGTGGCAGCGGCAGCGAGGTGTACTATCCT 2691
     LeuGlyLysIleProAlaThrAspPheAspAlaLeuIleCysGlySerGlySerGluValTyrTyrPro

2692 GGCACGGCGAACTGCATGGACGCTGAAGGAAAGCTGCGCCCAGATCAGGACTATCTGATGCACATCAGC 2760
     GlyThrAlaAsnCysMETAspAlaGluGlyLysLeuArgProAspGlnAspTyrLeuMETHisIleSer
                                                        4K

2761 CACCGCTGGTCCCATGACGGCGCGAGGCAGACCATAGCGAAGCTCATGGGCGCTCAGGACGGTTCAGGC 2829
     HisArgTrpSerHisAspGlyAlaArgGlnThrIleAlaLysLeuMETGlyAlaGlnAspGlySerGly
     ====================
         12N
```

FIGURE 7  3/4

35

```
2830 GACGCTGTCGAGCAGGACGTGGCGTCCAGTAATGCACACTGTGTCGCGTTCCTCATCAAAGACCCCCAA 2898
     AspAlaValGluGlnAspValAlaSerSerAsnAlaHisCysValAlaPheLeuIleLysAspProGln

2899 AAGGTGAAAACGGTCGATGAGATGAGGGAGCGGCTGAGGATGCGTGGTCTCCGCTGCCACATCATGTAC 2967
     LysValLysThrValAspGluMETArgGluArgLeuArgMETArgGlyLeuArgCysHisIleMETTyr

          PstI
          |
2968 TGCAGGAACTCGACAAGGCTTCAGGTTGTCCCTCTGCTAGCATCAAGGTCACAGGCACTCAGGTATCTT 3036
     CysArgAsnSerThrArgLeuGlnValValProLeuLeuAlaSerArgSerGlnAlaLeuArgTyrLeu
          2972

                                                                  XbaI
                                                                  |
3037 TCCGTGCGCTGGGGCGTATCTGTGGGGAACATGTATCTGATCACCGGGGAACATGGCGACACCGATCTA 3105
     SerValArgTrpGlyValSerValGlyAsnMETTyrLeuIleThrGlyGluHisGlyAspThrAspLeu
                                                                       3103

3106 GAGGAGATGCTATCCGGGCTACACAAGACCGTGATCGTCCGTGGCGTCACCGAGAAGGGTTCGGAAGCA 3174
     GluGluMETLeuSerGlyLeuHisLysThrValIleValArgGlyValThrGluLysGlySerGluAla

3175 CTGGTGAGGAGCCCAGGAAGCTACAAGAGGGACGATGTCGTCCCGTCTGAGACCCCCTTGGCTGCGTAC 3243
     LeuValArgSerProGlySerTyrLysArgAspAspValValProSerGluThrProLeuAlaAlaTyr

3244 ACGACTGGTGAGCTGAAGGCCGACGAGATCATGCGGGCTCTGAAGCAAGTCTCCAAGACTTCCAGCGGC 3312
     ThrThrGlyGluLeuLysAlaAspGluIleMETArgAlaLeuLysGlnValSerLysThrSerSerGly

3313 ATGTGAATTTGATGCTTCTTTTACATTTTGTCCTTTTCTTCACTGCTATATAAAATAAGTTGTGAACAG 3381
     MET .

3382 TACCGCGGGTGTGTATATATATATTGCAGTGACAAATAAAACAGGACACTGCTAACTATACTGGTGAAT 3450

                                           EcoRI
                                           |
3451 ATACGACTGTCAAGATTGTATGCTAAGTACTCCATTTCTCAATGTATCAATCGGAATTC 3509
                                                          3505
```

FIGURE 7  4/4

FIGURE 8

FIGURE 9